# EUROPEAN PATENT SPECIFICATION

(11) **EP 3 528 626 B1**
(45) Date of publication and mention of the grant of the patent: **31.08.2022**
(21) Application number: 17788159.6
(22) Date of filing: 10.10.2017
(51) Int. Cl.: A01N 25/00, A01N 25/08, A01N 25/26, A01N 59/00, A61K 9/46, A61K 9/14, A61K 31/165

(54) **USE OF SURFACE-REACTED CALCIUM CARBONATE FOR PREPARING SUPERSATURATED AQUEOUS SYSTEMS**
VERWENDUNG VON OBERFLÄCHENREAGIERTEM CALZIUMCARBONAT ZUR HERSTELLUNG ÜBERSÄTTIGTER WÄSSRIGER SYSTEME
UTILISATION DE CARBONATE DE CALCIUM TRAITÉ PAR RÉACTION DE SURFACE POUR LA PRÉPARATION DE SYSTÈMES AQUEUX SURSATURÉS

(30) Priority: 21.10.2016 EP 16195182; 28.10.2016 US 201662414041 P
(43) Date of publication of application: 28.08.2019
(73) Proprietor: Omya International AG, 4665 Oftringen (CH)
(72) Inventor: DIAZ QUIJANO, Carolina, 4665 Oftringen (CH); SCHENKER, Michel, 5012 Schönenwerd (CH); SCHOELKOPF,Joachim, 5727 Oberkulm (CH); GANE, Patrick A.C., 4852 Rothrist (CH)
(74) Representative: Glas, Holger
(86) International application number: PCT/EP2017/075743
(87) International publication number: WO 2018/073040

(56) References cited:
- WO-A1-2008/022745
- WO-A1-2016/113289
- WO-A1-2016/150773
- US-B2- 6 641 838
- ALAM MOHD AFTAB ET AL: "Effervescence Assisted Fusion Technique to Enhance the Solubility of Drugs", AAPS PHARMSCITECH, SPRINGER US, NEW YORK, vol. 16, no. 6, 12 August 2015 (2015-08-12), pages 1487-1494, XP036028905, DOI: 10.1208/S12249-015-0381-2
- PATRICK AUGUSTIJNS ET AL: "Supersaturating Drug Delivery Systems: Fast is Not Necessarily Good Enough", JOURNAL OF PHARMACEUTICAL SCIENCES, vol. 101, no. 1, 1 January 2012 (2012-01-01), pages 7-9, XP055357098, WASHINGTON, US ISSN: 0022-3549, DOI: 10.1002/jps.22750
- Y Zarga ET AL: "Calcium Sulfate and Calcium Carbonate Simple and Mixed Precipitations", Journal of New Sciences Volume, vol. 8, no. 01, 1 August 2014 (2014-08-01) , pages 7-16, XP055623155,

## Description

The present application relates to the use of surface-reacted calcium carbonate as a particulate carrier for preparing a supersaturated aqueous system comprising an active ingredient in dissolved state having a low water solubility.

Active ingredients are biologically active substances which are responsible for the efficacy of most human or veterinary medicines and pesticides.

Major efforts have been made to enhance the efficacy of active ingredients or the efficacy of corresponding formulations. Apart from the synthesis of novel active ingredients, the provision of synergistic compositions represents another way to enhance the efficacy of a given active ingredient, meaning that a combination of two or more such substances produces an effect greater than the sum of their individual effects caused by these substances. Apart from that, the combination of two or more active ingredients may allow for the prevention of new resistances, for example in the field of plant fungicides.

However, the efficacy of active ingredients or corresponding formulations is often limited by the bioavailability of the active ingredient itself. In many cases, active ingredients have a low solubility in water and are thus only poorly available in the target system under the conditions under which they are applied, for example under physiological conditions in human or animal bodies or under open-field conditions in the case of pesticides or plant nutrients.

The development of tailor-made formulations therefore represents a major principle in order to increase the effective amount of poorly water-soluble active ingredients within a given system and a given time period. Many of these formulations are designed to provide a sustained-release profile in order to prolong the efficacy. For example, US 2012/0295790 A1 relates to a pesticidal composition comprising sustained-release microcapsules which contain a pesticidal active ingredient and a suitable carrier and to a method of controlling pests comprising the application of an effective amount of such a pesticidal composition within a locus where pests are or are expected to be present. WO 2010/037753 A1 discloses a controlled-release active agent carrier, wherein said carrier comprises a surface-reacted natural or synthetic calcium carbonate and one or more active agents. P. Augustijns et al., Journal of Pharmaceutical Sciences, 2012, 101 (1), 7-9, describes the benefits of using supersaturation of active ingredients in drug delivery systems.

The international application published as WO 2016/113289 A1 relates to the use of surface-reacted calcium carbonate (SRCC) as a solid particulate carrier to enhance the efficacy of an agrochemical compound, especially the fungicides metalaxyl or dimethomorph, loaded onto said carrier alone or in combination with a copper source. Furthermore, WO 2016/113289 A1 also relates to a process for enhancing the efficacy of an agrochemical compound as well as to a process for the preparation of a corresponding agrochemical composition.

Some prior art documents are addressing possible relationships between the efficacy of active substances loaded onto carriers and the used carrier materials. For example, a change in the crystallization and dissolution behaviour of inherently crystalline active substances is proposed which, in turn, may result in an increased dissolution rate. In this connection, reference is made to the following publications: J. Forsgren et al., Adv. Healthcare Mater. 2013, 2, 1469 - 1476; D. Preisig et al., Eur. J. Pharm. Biopharm. 2014, 87, 548 - 558; D. Haid, "Functionalized Calcium Carbonate (FCC) as Drug Carrier", Master's Thesis, Spring 2013, University of Basel (Dpt. of Pharm. Sciences).

However, it would be desirable to further increase the effective amount of poorly water-soluble active ingredients in a given system, in particular of such active ingredients having a solubility limit in water of less than 10 g/l, measured at 20 °C and 1 bar.

In this respect, one object of the present invention may be seen in the provision of a formulation which provides an increased effective amount of a poorly water-soluble active ingredient compared with the active ingredient alone or the active ingredient present in conventional formulations.

One further object may be seen in the provision of a formulation providing a higher concentration of dissolved poorly water-soluble active ingredient compared with the same active ingredient applied alone or as a conventional formulation under identical conditions.

Still another object may be seen in the provision of a formulation containing an active ingredient, wherein said formulation may be applied less frequently and/or at lower overall dosage without significantly affecting the overall performance.

The foregoing and other problems may be solved by the subject-matter as defined herein in the independent claims.

A first aspect of the invention disclosed herein relates to the use of a loaded particulate carrier comprising surface-reacted calcium carbonate loaded with an active ingredient, said active ingredient having a solubility limit in water of less than 10 g/l, measured at 20 °C and 1 bar,
characterized in that the loaded particulate carrier is used for preparing an aqueous system comprising said active ingredient in dissolved form, wherein the mass concentration of dissolved active ingredient in said aqueous system corresponds to a supersaturated state, wherein the surface-reacted calcium carbonate is a reaction product of ground natural calcium carbonate (GNCC) or precipitated calcium carbonate (PCC) treated with CO₂ and one or more H₃O⁺ ion donors, wherein the CO₂ is formed in situ by the H₃O⁺ ion donors treatment and/or is supplied from an external source.

The inventors surprisingly found that surface-reacted calcium carbonate may be used as a particulate carrier for preparing supersaturated aqueous systems comprising poorly water-soluble active ingredients when the surface-reacted calcium carbonate is loaded with said active ingredient. The poorly water-soluble active ingredient may have a solubility limit in water of below 10 g/l, measured at 20 °C and 1 bar. The surface-reacted calcium carbonate is a reaction product of calcium carbonate (e.g. ground natural calcium carbonate or precipitated calcium carbonate) treated with CO₂ and one or more H₃O⁺ ion donors, wherein the CO₂ is formed in situ by the

H₃O⁺ ion donors treatment and/or is supplied from an external source. The use of surface-reacted calcium carbonate as carrier for poorly water-soluble active ingredients thus allows for the provision of higher effective amounts of said active ingredient compared with the use of the active ingredient alone or with the use of said active in conventional formulations. Specifically, the inventive use of particulate surface-reacted calcium carbonate as a carrier allows for the provision of aqueous systems comprising poorly water-soluble active ingredients in dissolved form, wherein the mass concentration of dissolved active ingredient in said aqueous system corresponds to a supersaturated state. In other words, the inventors surprisingly managed to prepare aqueous systems which show a greater concentration of an active ingredient in dissolved state than would exist at equilibrium, meaning that the mass concentration of the active ingredients exceeds the solubility limit of said active ingredient under the given conditions.

Another aspect of the present invention relates to an aqueous system, obtainable by contacting water and a loaded particulate carrier comprising surface-reacted calcium carbonate loaded with an active ingredient, said active ingredient having a solubility limit in water of less than 10 g/l, measured at 20 °C and 1 bar;
characterized in that the aqueous system comprises said active ingredient in dissolved form, wherein the mass concentration of dissolved active ingredient in said aqueous system corresponds to a supersaturated state, wherein the surface-reacted calcium carbonate is a reaction product of ground natural calcium carbonate (GNCC) or precipitated calcium carbonate (PCC) treated with CO₂ and one or more H₃O⁺ ion donors, wherein the CO₂ is formed in situ by the H₃O⁺ ion donors treatment and/or is supplied from an external source.

Still another aspect of the present invention relates to a method for preparing an aqueous system comprising an active ingredient in dissolved form, the method comprising the steps of:
(a) providing surface-reacted calcium carbonate;
(b) providing an active ingredient having a solubility limit in water of less than 10 g/l, measured at 20 °C and 1 bar;
(c) loading the surface-reacted calcium carbonate provided in step (a) with the active ingredient provided in step (b) to obtain a loaded particulate carrier;
(d) providing water;
(e) contacting the loaded particulate carrier obtained in step (c) and the water provided in step (d); and
(f) mixing the loaded particulate carrier and the water contacted in step (e);
characterized in that the mass concentration of dissolved active ingredient in said aqueous system corresponds to a supersaturated state, wherein the surface-reacted calcium carbonate is a reaction product of ground natural calcium carbonate (GNCC) or precipitated calcium carbonate (PCC) treated with CO₂ and one or more H₃O⁺ ion donors, wherein the CO₂ is formed in situ by the H₃O⁺ ion donors treatment and/or is supplied from an external source.

The following terms used throughout the present application shall have the meanings set forth hereinafter:
A "surface-reacted calcium carbonate" according to the present invention is a reaction product of ground natural calcium carbonate (GNCC) or precipitated calcium carbonate (PCC) treated with CO₂ and one or more H₃O⁺ ion donors, wherein the CO₂ is formed in situ by the H₃O⁺ ion donors treatment and/or is supplied from an external source. A H₃O⁺ ion donor in the context of the present invention is a Bronsted acid and/or an acid salt.

The term "ground natural calcium carbonate" (GNCC) as used herein refers to a particulate material obtained from natural calcium carbonate-containing minerals (e.g. chalk, limestone, marble or dolomite) which has been processed in a wet and/or dry comminution step, such as crushing and/or grinding, and optionally has been subjected to further steps such as screening and/or fractionation, for example, by a cyclone or classifier.

A "precipitated calcium carbonate" (PCC) in the meaning of the present invention is a synthesized material, generally obtained by precipitation following a reaction of carbon dioxide and calcium hydroxide (hydrated lime) in an aqueous environment or by precipitation of a calcium- and a carbonate source in water. Additionally, precipitated calcium carbonate can also be the product of introducing calcium- and carbonate salts, for example calcium chloride and sodium carbonate, in an aqueous environment. PCC may have a vateritic, calcitic or aragonitic crystalline form. PCCs are described, for example, in EP 2 447 213 A1, EP 2 524 898 A1, EP 2 371 766 A1, EP 2 840 065 A1, or WO 2013/142473 A1.

The term "supersaturated" as used herein refers to the physical state of a solution or system comprising at least one solvent (e.g. water) and at least one solute (e.g. dissolved active ingredient), wherein said solution or system shows a higher mass concentration of said solute being in dissolved state than would exist at equilibrium.

As used herein, the "solubility limit" of a specific solute is the mass concentration of said solute being in dissolved state within a saturated solution or system of a given solvent (e.g. water) and under given conditions, preferably at 20 °C and 1 bar. Where reference is made to the solubility limit in water, deionized water may be preferred. A "saturated solution" or "saturated system" in the meaning of the present application is understood to have the same concentration of a specific solute in dissolved state as one that is in equilibrium with undissolved solute under identical conditions (solvent, temperature, pressure etc.).

A "solution" as referred to herein is understood to be a single phase mixture of a specific solvent and a specific solute, for example a single phase mixture of an active ingredient and water. The term "dissolved" as used herein thus refers to the physical state of a solute in a solution.

"Water-insoluble" materials (e.g. water-insoluble calcium salts) are defined as materials which, when 100 g of said material is mixed with 100 g deionized water and filtered on a filter having a 0.2 µm pore size at 20 °C under atmospheric pressure to recover the liquid filtrate, provide less than or equal to 1 g of recovered solid material following evaporation at 95 to 100 °C of 100 g of said liquid filtrate at ambient pressure.

The term "particulate" in the meaning of the present application refers to materials composed of a plurality of particles. Said plurality of particles may be defined, for example, by its particle size distribution (*d*₉₈, *d*₅₀ etc.).

The "particle size" of surface-reacted calcium carbonate herein is described as volume-based particle size distribution *dₓ*(vol). Therein, the value *dₓ*(vol) represents the diameter relative to which *x* % by volume of the particles have diameters less than *dₓ*(vol). This means that, for example, the *d*₂₀(vol) value is the particle size at which 20 vol% of all particles are smaller than that particle size. The *d*₅₀(vol) value is thus the volume median particle size, i.e. 50 vol% of all particles are smaller than that particle size and the *d*₉₈(vol) value, referred to as volume top cut, is the particle size at which 98 vol% of all particles are smaller than that particle size.

The "particle size" of particulate materials other than surface-reacted calcium carbonate herein is described by its distribution of particle sizes *dₓ*(wt). Therein, the value *dₓ*(wt) represents the diameter relative to which *x* % by weight of the particles have diameters less than *dₓ*(wt). This means that, for example, the *d*₂₀(wt) value is the particle size at which 20 wt% of all particles are smaller than that particle size. The *d*₅₀(wt) value is thus the weight median particle size, i.e. 50 wt% of all particles are smaller than that particle size and the *d*₉₈(wt) value, referred to as weight top cut, is the particle size at which 98 wt% of all particles are smaller than that particle size.

An "active ingredient" in the meaning of the present application is understood to be a chemical compound which causes a specific biological activity when applied to a target organism (e.g. human body, animal body or plant). The term active ingredient as used herein thus includes both active forms and inactive precursors (prodrugs).

Throughout the present document, the "specific surface area" (in m²/g) of surface-reacted calcium carbonate or other materials is determined using the BET method (using nitrogen as adsorbing gas).

For the purpose of the present invention the "porosity" or "pore volume" refers to the intra-particle intruded specific pore volume.

In the context of the present invention, the term "pore" is to be understood as describing the space that is found between and/or within particles, i.e. that is formed by the particles as they pack together under nearest neighbour contact (interparticle pores), such as in a powder or a compact and/or the void space within porous particles (intraparticle pores), and that allows the passage of liquids under pressure when saturated by the liquid and/or supports absorption of surface wetting liquids.

A "suspension" or "slurry" in the meaning of the present invention refers to a mixture comprising at least one insoluble solid in a liquid medium, for example water, and optionally further additives, and usually contains large amounts of solids and, thus, is more viscous (higher viscosity) and can have a higher density than the liquid medium from which it is formed.

The term "solid" according to the present invention refers to a material that is solid under standard ambient temperature and pressure (SATP) which refers to a temperature of 298.15 K (25 °C) and an absolute pressure of exactly 1 bar. The solid may be in the form of a powder, tablet, granules, flakes etc.

A "carrier" in the meaning of the present application is to be understood as a substance which may be loaded with a second substance (e.g. a poorly water-soluble active ingredient) for the purpose of transporting said second substance to a target environment.

Unless specified otherwise, the term "drying" refers to a process according to which water is removed from a material to be dried such that a constant weight of the obtained "dried" material at 120 °C is reached, wherein the mass (sample size 5 g) does not change more than 1 mg over a period of 30 s.

Where an indefinite or definite article is used when referring to a singular noun, e.g. "a", "an" or "the", this includes a plural of that noun unless anything else is specifically stated.

Where the term "comprising" is used in the present description and claims, it does not exclude other elements. For the purposes of the present invention, the term "consisting of' is considered to be a preferred embodiment of the term "comprising". If hereinafter a group is defined to comprise at least a certain number of embodiments, this is also to be understood to disclose a group, which preferably consists only of these embodiments.

Terms like "obtainable" or "definable" and "obtained" or "defined" are used interchangeably. This, for example, means that, unless the context clearly dictates otherwise, the term "obtained" does not mean to indicate that, for example, an embodiment must be obtained by, for example, the sequence of steps following the term "obtained" though such a limited understanding is always included by the terms "obtained" or "defined" as a preferred embodiment.

Whenever the terms "including" or "having" are used, these terms are meant to be equivalent to "comprising" as defined hereinabove.

Advantageous embodiments of the inventive use of the particulate carrier are defined in the corresponding dependent claims.

The surface-reacted calcium carbonate is a reaction product of ground natural calcium carbonate (GNCC) or precipitated calcium carbonate (PCC) treated with CO₂ and one or more H₃O⁺ ion donors, wherein the CO₂ is formed in situ by the H₃O⁺ ion donors treatment and/or is supplied from an external source.

In another embodiment the one or more H₃O⁺ ion donor is selected from a strong acid, medium-strong acid, weak acid, or acidic salts thereof or mixtures thereof, wherein a strong acid has a pKa of 0 or less, a medium-strong acid has a pKa from 0 to 2.5, and a weak acid has a pKa greater than 2.5 and less or equal to 7, when measured at 20 °C, associated with the ionisation of the first available hydrogen, or acidic salts thereof, or mixtures thereof.

The surface-reacted calcium carbonate may be obtained by a process comprising the steps of:
(a) providing a suspension of natural or precipitated calcium carbonate,
(b) adding at least one acid having a pKₐ value of 0 or less at 20 °C or having a pKₐ value from 0 to 2.5 at 20 °C to the suspension of step (a); and
(c) treating the suspension of step (a) with carbon dioxide before, during
   or after step (b).

Said acid having a pKₐ value of 0 or less at 20 °C may be selected from sulphuric acid, hydrochloric acid or mixtures thereof.

Said acid having a pKₐ value from 0 to 2.5 at 20 °C, the acid may be selected from H₂SO₃, H₃PO₄, oxalic acid or mixtures thereof.

The surface-reacted calcium carbonate may be obtained by a process comprising the steps of:
(a) providing a ground natural calcium carbonate (GNCC) or precipitated calcium carbonate (PCC);
(b) providing at least one acid;
(c) providing gaseous CO₂; and
(d) contacting said GNCC or PCC provided in step (a), the at least one acid provided in step (b) and the gaseous CO₂ provided in step (c);
   characterized in that
   (i) the at least one acid provided in step (b) has a pKₐ of greater than 2.5 and less than or equal to 7 at 20 °C, associated with the ionisation of its first available hydrogen, and a corresponding anion is formed on loss of this first available hydrogen capable of forming a water-soluble calcium salt; and
   (ii) following contacting the at least one water-soluble acid provided in step (b) and the GNCC or PCC provided in step (a), at least one water-soluble salt, which in the case of a hydrogen-containing salt has a pKₐ of greater than 7 at 20 °C, associated with the ionisation of the first available hydrogen, and the salt anion of which is capable of forming water-insoluble calcium salts, is additionally provided.

In still another embodiment according to the present invention, the surface-reacted calcium carbonate has:
(i) a specific surface area of from 15 m²/g to 200 m²/g, preferably from 27 m²/g to 180 m²/g, more preferably from 30 m²/g to 160 m²/g, even more preferably from 45 m²/g to 150 m²/g, most preferably from 48 m²/g to 140 m²/g, measured using nitrogen and the BET method. measured using nitrogen and the BET method according to ISO 9277:2010;
(ii) a volume median grain diameter *d*₅₀(vol) of from 1 to 75 µm, preferably from 2 to 50 µm, more preferably 3 to 40 µm, even more preferably from 4 to 30 µm, and most preferably from 5 to 15 µm;
(iii) a grain diameter *d*₉₈(vol) of from 2 to 150 µm, preferably from 4 to 100 µm, more preferably 6 to 80 µm, even more preferably from 8 to 60 µm, and most preferably from 10 to 30 µm; and/or
(iv) an intra-particle intruded specific pore volume in the range from 0.1 to 2.3 cm³/g, more preferably from 0.2 to 2.0 cm³/g, especially preferably from 0.4 to 1.8 cm³/g and most preferably from 0.6 to 1.6 cm³/g, calculated from mercury porosimetry measurement.

In another embodiment, the active ingredient has a solubility limit in water, measured at 20 °C and 1 bar, of less than 5 g/l, preferably less than 1 g/l, and most preferably less than 0.1 g/l.

In still another embodiment, the surface-reacted calcium carbonate is used in a weight ratio of from 100:1 to 1:10, preferably 50:1 to 1:2, and most preferably 20:1 to 1:1 on a dry weights basis relative to the weight of the active ingredient.

In still another embodiment of the present invention, the loaded particulate carrier is used in an amount such that the theoretical mass concentration of dissolved active ingredient in the aqueous system is at most 10 times, preferably at most 5 times, and most preferably at most 3 times higher than the solubility limit of said active ingredient under identical conditions.

According to another embodiment, the mass concentration of dissolved active ingredient in the aqueous system is at least 1.1 times, preferably at least 1.5 times, and most preferably at least 2 times higher than the solubility limit of said active ingredient under identical conditions.

According to still another embodiment, the aqueous system has a pH value in the range of from 1.5 to 10, preferably from 3 to 9, more preferably from 4 to 8 and most preferably from 6.5 to 7.5.

In still another embodiment, the active ingredient is selected from pharmaceutical drugs, agrochemical compounds including pesticides and fertilizers, biocides, micronutrients, antimicrobial agents including antifungal agents and antibacterial agents, and mixtures thereof; preferably the biocide is selected from the group consisting of phenols, halogenated phenols, halogen-containing compounds, halogen-releasing compounds, isothiazolinones, aldehyde-containing compounds, aldehyde-releasing compounds, biguanides, sulfones, thiocyanates, pyrithiones, antibiotics such as β-lactam antibiotics, quaternary ammonium salts, peroxides, perchlorates, amides, amines, heavy metals, biocidal enzymes, biocidal polypeptides, azoles, carbamates, glyphosates, sulphonamides and mixtures thereof; more preferably the active ingredient is selected from L-carvone, chloramphenicol, curcumin, 2-phenylphenol, vanillin and mixtures thereof; most preferably the active compound is chloramphenicol or 2-phenylphenol.

In the following, preferred embodiments of the inventive use of the loaded particulate carrier will be discussed in more detail. It is to be understood that these details and embodiments also apply to the inventive aqueous system as well as to the inventive process for the preparation of said aqueous system.

### (A) Surface-reacted calcium carbonate

The particulate carrier used in the present invention is a surface-reacted calcium carbonate. Surface-reacted calcium carbonate is also referred to as functionalized calcium carbonate (FCC).

It is appreciated that the surface-reacted calcium carbonate can be one or a mixture of different kinds of surface-reacted calcium carbonate(s). In one embodiment of the present invention, the surface-reacted calcium carbonate comprises, preferably consists of, one kind of surface-reacted calcium carbonate. Alternatively, the surface-reacted calcium carbonate comprises, preferably consists of, two or more kinds of surface-reacted calcium carbonates. For example, the surface-reacted calcium carbonate comprises, preferably consists of, two or three kinds of surface-reacted calcium carbonates. Preferably, the surface-reacted calcium carbonate comprises, more preferably consists of, one kind of surface-reacted calcium carbonate.

The surface-reacted calcium carbonate is a reaction product of ground natural calcium carbonate (GNCC) or precipitated calcium carbonate (PCC) treated with CO₂ and one or more H₃O⁺ ion donors, wherein the CO₂ is formed in situ by the H₃O⁺ ion donors treatment and/or is supplied from an external source. Because of the reaction of ground natural calcium carbonate or precipitated calcium carbonate with CO₂ and the one or more H₃O⁺ ion donors, surface-reacted calcium carbonate may comprise GNCC or PCC and at least one water-insoluble calcium salt.

In a preferred embodiment, said surface-reacted calcium carbonate comprises GNCC or PCC and at least one water-insoluble calcium salt which is present on at least part of the surface of said GNCC or PCC.

An H₃O⁺ ion donor in the context of the present invention is a Bronsted acid and/or an acid salt.

In a preferred embodiment of the invention, the surface-reacted calcium carbonate is obtained by a process comprising the steps of:
(a) providing a suspension of ground natural calcium carbonate (GNCC) or precipitated calcium carbonate (PCC);
(b) adding at least one acid having a pKₐ value of 0 or less at 20 °C, or having a pKₐ value from 0 to 2.5 at 20 °C to the suspension provided in step (a); and
(c) treating the suspension provided in step (a) with CO₂ before, during or after step (b).

According to another embodiment, the surface-reacted calcium carbonate is obtained by a process comprising the steps of:
(a) providing a ground natural calcium carbonate (GNCC) or precipitated calcium carbonate (PCC);
(b) providing at least one water-soluble acid;
(c) providing gaseous CO₂; and
(d) contacting said GNCC or PCC provided in step (a), the at least one acid provided in step (b) and the gaseous CO₂ provided in step (c);
   characterized in that
   (i) the at least one acid provided in step (b) has a pKₐ of greater than 2.5 and less than or equal to 7 at 20 °C, associated with the ionisation of its first available hydrogen, and a corresponding anion is formed on loss of this first available hydrogen capable of forming a water-soluble calcium salt; and
   (ii) following contacting the at least one water-soluble acid provided in step (b) and the GNCC or PCC provided in step (a), at least one water-soluble salt, which in the case of a hydrogen-containing salt has a pKₐ of greater than 7 at 20 °C, associated with the ionisation of the first available hydrogen, and the salt anion of which is capable of forming water-insoluble calcium salts, is additionally provided.

The source of calcium carbonate, e.g. ground natural calcium carbonate (GNCC), preferably is selected from calcium carbonate-containing minerals selected from the group comprising marble, chalk, limestone and mixtures thereof. Natural calcium carbonate may comprise further naturally occurring components such as magnesium carbonate, alumino silicate etc. According to one embodiment, natural calcium carbonate, such as GNCC, comprises aragonitic, vateritic or calcitic mineralogical crystal forms of calcium carbonate or mixtures thereof.

In general, the grinding of ground natural calcium carbonate may be performed in a dry or wet grinding process and may be carried out with any conventional grinding device, for example, under conditions such that comminution predominantly results from impacts with a secondary body, i.e. in one or more of: a ball mill, a rod mill, a vibrating mill, a roll crusher, a centrifugal impact mill, a vertical bead mill, an attrition mill, a pin mill, a hammer mill, a pulverizer, a shredder, a de-clumper, a knife cutter, or other such equipment known to the skilled person. In case the ground natural calcium carbonate comprises wet ground calcium carbonate, the grinding step may be performed under conditions such that autogenous grinding takes place and/or by horizontal ball milling, and/or other such processes known to the skilled person. The wet processed ground natural calcium carbonate thus obtained may be washed and dewatered by well-known processes, e.g. by flocculation, filtration or forced evaporation prior to drying. The subsequent step of drying (if necessary) may be carried out in a single step such as spray drying, or in at least two steps. It is also common that such a mineral material undergoes a beneficiation step (such as a flotation, bleaching or magnetic separation step) to remove impurities.

As already indicated hereinabove, a precipitated calcium carbonate (PCC) in the meaning of the present invention is a synthesized material, generally obtained by precipitation following a reaction of CO₂ and calcium hydroxide in an aqueous environment or by precipitation of calcium and carbonate ions, for example CaCl₂ and Na₂CO₃, out of solution. Further possible ways of producing PCC are the lime soda process, or the Solvay process in which PCC is a by-product of ammonia production. Precipitated calcium carbonate exists in three primary crystalline forms: calcite, aragonite and vaterite, and there are many different polymorphs (crystal habits) for each of these crystalline forms. Calcite has a trigonal structure with typical crystal habits such as scalenohedral (S-PCC), rhombohedral (R-PCC), hexagonal prismatic, pinacoidal, colloidal (C-PCC), cubic, and prismatic (P-PCC). Aragonite is an orthorhombic structure with typical crystal habits of twinned hexagonal prismatic crystals, as well as a diverse assortment of thin elongated prismatic, curved bladed, steep pyramidal, chisel shaped crystals, branching tree, and coral or worm-like form. Vaterite belongs to the hexagonal crystal system. The obtained aqueous PCC slurry can be mechanically dewatered and dried.

According to one embodiment of the present invention, the precipitated calcium carbonate comprises aragonitic, vateritic or calcitic mineralogical crystal forms of calcium carbonate or mixtures thereof.

Precipitated calcium carbonate may be ground prior to the treatment with CO₂ and at least one H₃O⁺ ion donor by the same means as used for grinding natural calcium carbonate and described above.

According to one embodiment of the present invention, the natural or precipitated calcium carbonate is in form of particles having a weight median particle size *d*₅₀(wt) of from 0.05 to 10.0 µm, preferably from 0.2 to 5.0 µm, more preferably from 0.4 to 3.0 µm, most preferably from 0.6 to 1.2 µm, and especially 0.7 µm. According to a further embodiment of the present invention, the natural or precipitated calcium carbonate is in form of particles having a top cut particle size *d*₉₈(wt) of from 0.15 to 55 µm, preferably from 1 to 40 µm, more preferably from 2 to 25 µm, most preferably from 3 to 15 µm, and especially 4 µm.

The natural or precipitated calcium carbonate may be used dry or suspended in water. Preferably, a corresponding aqueous slurry has a content of natural or precipitated calcium carbonate within the range of from 1 to 90 wt%, more preferably from 3 to 60 wt%, even more preferably from 5 to 40 wt%, and most preferably from 10 to 25 wt%, based on the total weight of said slurry.

The one or more H₃O⁺ ion donor used for the preparation of surface reacted calcium carbonate may be any strong acid, medium-strong acid, or weak acid, or mixtures thereof, generating H₃O⁺ ions under the preparation conditions. According to the present invention, the at least one H₃O⁺ ion donor can also be an acid salt, generating H₃O⁺ ions under the preparation conditions.

According to one embodiment, the at least one H₃O⁺ ion donor is a strong acid having a pKₐ of 0 or less at 20 °C.

According to another embodiment, the at least one H₃O⁺ ion donor is a medium-strong acid having a pKₐ value from 0 to 2.5 at 20 °C. If the pKₐ at 20 °C is 0 or less, the acid is preferably selected from sulphuric acid, hydrochloric acid, or mixtures thereof. If the pKₐ at 20 °C is from 0 to 2.5, the H₃O⁺ ion donor is preferably selected from H₂SO₃, H₃PO₄, oxalic acid, or mixtures thereof. The at least one H₃O⁺ ion donor can also be an acid salt, for example, HSO₄⁻ or H₂PO₄⁻, being at least partially neutralized by a corresponding cation such as Li⁺, Na⁺ or K⁺, or HPO₄²⁻, being at least partially neutralized by a corresponding cation such as Li⁺, Na^{+,} K⁺, Mg²⁺ or Ca²⁺. The at least one H₃O⁺ ion donor can also be a mixture of one or more acids and one or more acid salts.

According to still another embodiment, the at least one H₃O⁺ ion donor is a weak acid having a pKₐ value of greater than 2.5 and less than or equal to 7, when measured at 20 °C, associated with the ionisation of the first available hydrogen, and having a corresponding anion, which is capable of forming water-soluble calcium salts. Subsequently, at least one water-soluble salt, which in the case of a hydrogen-containing salt has a pKₐ of greater than 7, when measured at 20 °C, associated with the ionisation of the first available hydrogen, and the salt anion of which is capable of forming water-insoluble calcium salts, is additionally provided. According to a more preferred embodiment, the weak acid has a pKₐ value from greater than 2.5 to 5 at 20 °C, and more preferably the weak acid is selected from the group consisting of acetic acid, formic acid, propanoic acid and mixtures thereof. Exemplary cations of said water-soluble salt are selected from the group consisting of potassium, sodium, lithium and mixtures thereof. In a more preferred embodiment, said cation is sodium or potassium. Exemplary anions of said water-soluble salt are selected from the group consisting of phosphate, dihydrogen phosphate, monohydrogen phosphate, oxalate, silicate, mixtures thereof and hydrates thereof. In a more preferred embodiment, said anion is selected from the group consisting of phosphate, dihydrogen phosphate, monohydrogen phosphate, mixtures thereof and hydrates thereof. In a most preferred embodiment, said anion is selected from the group consisting of dihydrogen phosphate, monohydrogen phosphate, mixtures thereof and hydrates thereof. Water-soluble salt addition may be performed dropwise or in one step. In the case of dropwise addition, this addition preferably takes place within a time period of 10 min. It is more preferred to add said salt in one step.

According to one embodiment of the present invention, the at least one H₃O⁺ ion donor is selected from the group consisting of hydrochloric acid, sulphuric acid, sulphurous acid, phosphoric acid, citric acid, oxalic acid, acetic acid, formic acid and mixtures thereof. Preferably the at least one H₃O⁺ ion donor is selected from the group consisting of hydrochloric acid, sulphuric acid, sulphurous acid, phosphoric acid, oxalic acid, H₂PO₄⁻, being at least partially neutralized by a corresponding cation such as Li⁺, Na⁺ or K⁺, HPO₄²⁻, being at least partially neutralized by a corresponding cation such as Li⁺, Na⁺, K⁺, Mg²⁺ or Ca²⁺ and mixtures thereof, more preferably the at least one acid is selected from the group consisting of hydrochloric acid, sulphuric acid, sulphurous acid, phosphoric acid, oxalic acid, or mixtures thereof. A particularly preferred H₃O⁺ ion donor is phosphoric acid.

The one or more H₃O⁺ ion donor can be added to the suspension as a concentrated solution or a more diluted solution. Preferably, the molar ratio of the H₃O⁺ ion donor to the natural or precipitated calcium carbonate is from 0.01 to 4, more preferably from 0.02 to 2, even more preferably from 0.05 to 1 and most preferably from 0.1 to 0.58.

In another preferred embodiment, the at least one H₃O⁺ ion donor is selected from the group consisting of hydrochloric acid, sulphuric acid, sulphurous acid, phosphoric acid, citric acid, oxalic acid, acetic acid, formic acid and mixtures thereof, wherein the molar ratio of the H₃O⁺ ion donor to the natural or precipitated calcium carbonate is from 0.01 to 4, more preferably from 0.02 to 2, even more preferably from 0.05 to 1 and most preferably from 0.1 to 0.58.

In a particularly preferred embodiment, the at least one H₃O⁺ ion donor is a mixture of phosphoric acid and citric acid, more preferably the molar ratio of the H₃O⁺ ion donor to the natural or precipitated calcium carbonate is from 0.01 to 4, more preferably from 0.02 to 2, even more preferably from 0.05 to 1 and most preferably from 0.1 to 0.58.

As already indicated hereinabove, the treatment of GNCC or PCC with the at least one H₃O⁺ ion donor and CO₂ may lead to the formation of at least one water-insoluble calcium salt. Therefore, surface-reacted calcium carbonate may comprise GNCC or PCC and at least one water-insoluble calcium salt other than calcium carbonate. In one embodiment, said at least one water-insoluble calcium salt is present on at least part of the surface of said GNCC or PCC.

The use of phosphoric acid, H₂PO₄⁻ or HPO₄²⁻ as the H₃O⁺ ion donor may lead to the formation of hydroxylapatite. Therefore, in a preferred embodiment, the at least one water-insoluble calcium salt is hydroxylapatite.

The amount of the at least one water-insoluble calcium salt present in the surface-reacted calcium carbonate may be quantified by XRD relative to the amount of calcite, aragonite and/or vaterite which is present in GNCC or PCC using the Rietveld method.

In a more preferred embodiment, the at least one water-insoluble calcium salt is hydroxylapatite, wherein the surface-reacted calcium carbonate provides a ratio of hydroxylapatite to calcite, aragonite and/or vaterite, preferably to calcite, in the range of from 1:99 to 99:1 by weight. Still more preferably, the surface-reacted calcium carbonate provides a ratio of hydroxylapatite to calcite, aragonite and/or vaterite, preferably to calcite, in the range of from 1:9 to 9:1, preferably 1:7 to 8:1, more preferably 1:5 to 7:1 and most preferably 1:4 to 7:1 by weight.

In a similar manner, the use of other H₃O⁺ ion donors may lead to the formation of corresponding water-insoluble calcium salts other than calcium carbonate on at least part of the surface of the surface-reacted calcium carbonate. In one embodiment, the at least one water-insoluble calcium salt is thus selected from the group consisting of octacalcium phosphate, hydroxylapatite, chlorapatite, fluorapatite, carbonate apatite and mixtures thereof, wherein the surface-reacted calcium carbonate shows a ratio of the at least one water-insoluble calcium salt to calcite, aragonite and/or vaterite, preferably to calcite, in the range of from 1:99 to 99:1, preferably from 1:9 to 9:1, more preferably from 1:7 to 8:1, even more preferably from 1:5 to 7:1 and most preferably from 1:4 to 7:1 by weight.

As an alternative, it is also possible to add the H₃O⁺ ion donor to the water before the natural or precipitated calcium carbonate is suspended.

In a next step, the natural or precipitated calcium carbonate is treated with CO₂. If a strong acid such as sulphuric acid or hydrochloric acid is used for the H₃O⁺ ion donor treatment of the natural or precipitated calcium carbonate, the CO₂ is automatically formed. Alternatively or additionally, the CO₂ can be supplied from an external source.

H₃O⁺ ion donor treatment and treatment with CO₂ can be carried out simultaneously which is the case when a strong or medium-strong acid is used. It is also possible to carry out H₃O⁺ ion donor treatment first, e.g. with a medium strong acid having a pKₐ in the range of 0 to 2.5 at 20 °C, wherein CO₂ is formed in situ, and thus, the CO₂ treatment will automatically be carried out simultaneously with the H₃O⁺ ion donor treatment, followed by the additional treatment with CO₂ supplied from an external source.

Preferably, the concentration of gaseous CO₂ in the suspension is, in terms of volume, such that the ratio (volume of suspension): (volume of gaseous CO₂) is from 1:0.05 to 1:20, even more preferably 1:0.05 to 1:5.

In a preferred embodiment, the H₃O⁺ ion donor treatment step and/or the CO₂ treatment step are repeated at least once, more preferably several times. According to one embodiment, the at least one H₃O⁺ ion donor is added over a time period of at least about 5 min, preferably at least about 10 min, typically from about 10 to about 20 min, more preferably about 30 min, even more preferably about 45 min, and sometimes about 1 h or more.

Subsequent to the H₃O⁺ ion donor treatment and CO₂ treatment, the pH of the aqueous suspension, measured at 20 °C, naturally reaches a value of greater than 6.0, preferably greater than 6.5, more preferably greater than 7.0, even more preferably greater than 7.5, thereby preparing the surface-reacted natural or precipitated calcium carbonate as an aqueous suspension having a pH of greater than 6.0, preferably greater than 6.5, more preferably greater than 7.0, even more preferably greater than 7.5.

Further details about the preparation of the surface-reacted natural calcium carbonate are disclosed in WO 00/39222 A1, WO 2004/083316 A1, WO 2005/121257 A2, WO 2009/074492 A1, EP 2 264 108 A1, EP 2 264 109 A1 and US 2004/0020410 A1.

Similarly, surface-reacted precipitated calcium carbonate may be obtained. As can be taken in detail from WO 2009/074492 A1, surface-reacted precipitated calcium carbonate is obtained by contacting precipitated calcium carbonate with H₃O⁺ ions and with anions being solubilized in an aqueous medium and being capable of forming water-insoluble calcium salts, in an aqueous medium to form a slurry of surface-reacted precipitated calcium carbonate, wherein said surface-reacted precipitated calcium carbonate comprises an insoluble, at least partially crystalline calcium salt of said anion formed on the surface of at least part of the precipitated calcium carbonate.

Said solubilized calcium ions correspond to an excess of solubilized calcium ions relative to the solubilized calcium ions naturally generated on dissolution of precipitated calcium carbonate by H₃O⁺ ions, where said H₃O⁺ ions are provided solely in the form of a counter ion to the anion, i.e. via the addition of the anion in the form of an acid or non-calcium acid salt, and in absence of any further calcium ion or calcium ion generating source.

Said excess solubilized calcium ions are preferably provided by the addition of a soluble neutral or acid calcium salt, or by the addition of an acid or a neutral or acid non-calcium salt which generates a soluble neutral or acid calcium salt in situ.

Said H₃O⁺ ions may be provided by the addition of an acid or an acid salt of said anion, or the addition of an acid or an acid salt which simultaneously serves to provide all or part of said excess solubilized calcium ions.

In a further preferred embodiment of the preparation of the surface-reacted natural or precipitated calcium carbonate, the natural or precipitated calcium carbonate is reacted with the acid and/or the CO₂ in the presence of at least one compound selected from the group consisting of silicate, silica, aluminium hydroxide, earth alkali aluminate such as sodium or potassium aluminate, magnesium oxide, aluminium sulphate or mixtures thereof. Preferably, the at least one silicate is selected from an aluminium silicate, a calcium silicate, or an earth alkali metal silicate.

In another preferred embodiment, said at least one compound is aluminium sulphate hexadecahydrate. In a particularly preferred embodiment, said at least one compound is aluminium sulphate hexadecahydrate, wherein the at least one H₃O⁺ ion donor is selected from the group consisting of hydrochloric acid, sulphuric acid, sulphurous acid, phosphoric acid, citric acid, oxalic acid, acetic acid, formic acid and mixtures thereof, more preferably the molar ratio of said H₃O⁺ ion donor to the natural or precipitated calcium carbonate is from 0.01 to 4, more preferably from 0.02 to 2, even more preferably from 0.05 to 1 and most preferably from 0.1 to 0.58.

The foregoing components can be added to an aqueous suspension comprising the natural or precipitated calcium carbonate before adding the acid and/or CO₂.

Alternatively, the foregoing components can be added to the aqueous suspension of natural or precipitated calcium carbonate while the reaction of natural or precipitated calcium carbonate with an acid and CO₂ has already started. Further details about the preparation of the surface-reacted natural or precipitated calcium carbonate in the presence of at least one silicate and/or silica and/or aluminium hydroxide and/or earth alkali aluminate component(s) are disclosed in WO 2004/083316 A1, the content of this reference herewith being included in the present application.

The surface-reacted calcium carbonate can be kept in suspension, optionally further stabilized by a dispersant. Conventional dispersants known to the skilled person can be used. A preferred dispersant is comprised of polyacrylic acids and/or carboxymethylcelluloses.

Alternatively, the aqueous suspension described above can be dried, thereby obtaining the solid (i.e. dry or containing as little water that it is not in a fluid form) surface-reacted natural or precipitated calcium carbonate in the form of granules or a powder.

The surface reacted calcium carbonate may have different particle shapes, such as e.g. the shape of roses, golf balls and/or brains.

In a preferred embodiment, the surface-reacted calcium carbonate has a specific surface area of from 15 to 200 m²/g, preferably from 27 to 180 m²/g, more preferably from 30 to 160 m²/g, even more preferably from 45 to 150 m²/g, and most preferably from 48 to 140 m²/g, measured using nitrogen and the BET method according to ISO 9277:2010. In a further embodiment, the surface-reacted calcium carbonate has a specific surface area of 120 m²/g or less, more preferably from 60 to 120 m²/g, and most preferably from 70 to 105 m²/g, measured using nitrogen and the BET method according to ISO 9277:2010. For example, the surface-reacted calcium carbonate may have a specific surface area of from 75 to 100 m²/g, measured using nitrogen and the BET method according to ISO 9277:2010.

It may furthermore be preferred that the surface-reacted calcium carbonate particles have a volume median grain diameter *d*₅₀(vol) of from 1 to 75 µm, preferably from 2 to 50 µm, more preferably from 3 to 40 µm, even more preferably from 4 to 30 µm, and most preferably from 5 to 15 µm. According to another preferred embodiment, the surface-reacted calcium carbonate particles have a volume median grain diameter *d*₅₀(vol) of from 1.5 to 12 µm, preferably from 2 to 5 µm or from 6 to 10 µm.

It may furthermore be preferred that the surface-reacted calcium carbonate particles have a grain diameter *d*₉₈(vol) of from 2 to 150 µm, preferably from 4 to 100 µm, more preferably from 6 to 80 µm, even more preferably from 8 to 60 µm, and most preferably from 10 to 30 µm. According to another preferred embodiment, the surface-reacted calcium carbonate particles have a volume median grain diameter *d*₉₈(vol) of from 5 to 20 µm, preferably from 8 to 12 µm or from 13 to 18 µm. According to another embodiment, the surface-reacted calcium carbonate has an intra-particle intruded specific pore volume in the range from 0.1 to 2.3 cm³/g, more preferably from 0.2 to 2.0 cm³/g, especially preferably from 0.4 to 1.8 cm³/g and most preferably from 0.6 to 1.6 cm³/g, calculated from mercury porosimetry measurement.

The intra-particle pore size of the surface-reacted calcium carbonate preferably is in a range of from 0.004 to 1.6 µm, more preferably in a range of between 0.005 to 1.3 µm, especially preferably from 0.006 to 1.15 µm and most preferably of 0.007 to 1.0 µm, e.g. 0.004 to 0.50 µm determined by mercury porosimetry measurement.

### (B) Active ingredients

The surface-reacted calcium carbonate used in the present invention as a particulate carrier is loaded with an active ingredient.

As already indicated hereinabove, the active ingredient in the meaning of the present invention is a chemical compound which causes a specific biological activity when applied to a target organism (e.g. human body, animal body or plant). The term active ingredient as used herein includes both active forms and inactive precursors (prodrugs). In one embodiment, the active ingredient thus may be selected from active ingredients for use in human drugs and inactive precursors thereof, active ingredients for use in animal drugs and inactive precursors thereof as well as agrochemical compounds and inactive precursors thereof.

In a further embodiment, the active ingredient is an active ingredient for use in human and/or animal drugs. Preferably, said active ingredient is an antibiotic.

In still another embodiment of the present invention, the active ingredient is an agrochemical compound. The agrochemical compounds in the meaning of the present invention may be selected from pesticides, fertilizers including micronutrient fertilizers, soil additives and phytohormones. The pesticide may be selected from the group consisting of bactericides, fungicides, acaricides, insecticides, molluscicides, nematicides, rodenticides, avicides, and herbicides, and mixtures thereof. Preferably, the active ingredient is a fungicide, more preferably a post-harvest fungicide.

In still another embodiment of the present invention, the active ingredient is selected from the group consisting of phenols, halogenated phenols, halogen-containing compounds, halogen-releasing compounds, isothiazolinones, aldehyde-containing compounds, aldehyde-releasing compounds, biguanides, sulfones, thiocyanates, pyrithiones, β-lactams, quaternary ammonium salts, peroxides, perchlorates, amides, amines, heavy metals, biocidal enzymes, biocidal polypeptides, azoles, carbamates, glyphosates, sulphonamides and mixtures thereof.

Non-limiting examples of suitable active ingredients are iodopropargyl butyl carbamate (IPBC), benzisothiazolone (BIT), N-butyl-benzisothiazolinone (BBIT), propiconazole, N(trichloromethylthio)phthalimide (Folpet/Pestanal), Fluor-Folpet, methyl benzimidazol-2-yl carbamate (Carbendazim), tetrachloroisophalonitrile (chlorothalonil), 2,4-dichlorophenyl methanol, phenetylcarbinol, DCHB, 2-hydroxy-1-naphthaldehyde (HNA), 2-nitro-2-trifluoromethyl-1,3-propanediol, N-methylene-cyclohexylamine, 1,3-bis(2-ethlhexyl)-5-methyl-hexahydripyrimidin-5-yl-amine, hexetidine, 3-hydroxymethyl-5,6-dichloro-benzoxazolinone, 4-isopropyl-3-methylphenol, 5-isopropyl-2-methylphenol, 2-benzylphenol, 2-cyclohexylphenol, 2-chloro-5-hydroxy-1,3-dimethylbenzene (PCMX), DCMX, chlorthymol, chlorophen, 2-phenylphenol (OPP), 4-biphenylol, 4-chloro-2-hydroxybiphenyl, 4-chlorophenol, 2,4,5-trichlorophenol, pentachlorophenol (PCP), 2,4,6-tribromophenol, tinosan, bis-(4-hydroxyphenyl)-methane, 2,2-bis(4-hydroxyphenyl) propane, hexachlorophen, bromochlorophen, fentichlor, bithionol, 4-nitrophenol, caprylic acid, n-octanoic acid, undec-10-enoic acid, dehydroacetic acid (DHA), n-propyl 4-hydroxybenzoate, n-butyl 4-hydroxybenzoate, benzyl 4-hydroxybenzoate, naphthenic acid, ethyl formate, ethyl bromoacetate, benzyl bromoacetate, 1,2-bis(bromoacetoxy) ethane, 1,4-bis(bromoacetoxy)-2-butene (BBAB), 1-bromo-3-ethoxycarbonyloxy-1,2-diiodo-1-propene, glyceryl monolaurate (a- and b-form), pentachlorophenyl laurate, fatty acid esters of 5,50-dichloro-2,20-dihydroxydiphenylmethane, salicylamide, salicylanilide, tribromosalan (TBS), dithio-2,20-bis(benzmethylamide), furmecyclox, N-(2-methylnaphthyl)pyrrol-2,5-dione, triclocarban (TCC), diuron, 4-trifluoromethylphenylsulfonic acid amide, 3-iodopropynylphenylcarbamate (IPPC), 3-iodopropynylcarbamate (IPC), benomyl, 3-butyl-2,4-dioxo-s-triazino[1,2-a]benzimidazole (STB), 5,6-dichlorobenzoxazolinone, zinc dimethyldithiocarbamate, Zinc ethylenebisdithiocarbamate, thiram, zinc pyrithione, copper pyrithione, 8-quinolinol, copper 8-quinolinolate, tebuconazole, propiconazole, azaconazole, cyproconazole, 2-n-octyl-4-isothiazolin-3-one (OI), 4,5-dichloro-2-(n-octyl)-4-isothiazolin-3-one (DCOI), thiabendazole, MBT, 2-benzothiazolyl mercaptan, 2-(thiocyanomethylthio)benzthiazole (TCMBT), captan, captafol, dichlofluanide, tolylfluanide, alpha-bromoacetamide, alpha-iodoacetamide, BMPCA, 4-(bromacetyl)phenol, bis(trichloromethyl)sulphone, 4-chlorophenyl-diiodomethylsulphone, 1-chloro-1-cyano-2-phenylsulphonylethylene, methyl tetrachloropyridin-4-yl sulfone, (2-Bromo-2-nitroethenyl)-benzene, anilazine, dyrene, 4,5-dichloro-3-oxo-1,2-dithiole, dodecylamine, chlorhexidine, chlorhexidine dihydrochloride, phenylmercury (II) oleate, tributyltin benzoate (TBTB), (Z,Z)-tributyl(octadeca-9,12-dienoyl)stannane, tributyltin naphthenate (TBTN), tributyltin fluoride (TBTF), triphenyltin chloride (TPTC), bis-(N-cyclohexyldiazeniumdioxy)-copper (Cu-HDO), tridemorph, fenpropimorph, simazine, terbutylethylazine, 2-tert-butylamino-6-chloro-4-ethylamino-s-triazine, biphenyl, alpha-chloronaphthalene, allyl isothiocyanate, polymethacrylic acid tert-butylaminoethyl ester, nisin A, pimaricin, 6-(1,3-dioxo-1,3dihydro-isoindol-2-yl)-hexaneperoxoic acid, trichloromelamine (TCM), succinchlorimide, 2-n-octyl-3-isothiazolone, dibromo-nitriloproprianamide, 2-(thiocyanomethylthio) benzothiazole (TCMTB), tebuconazole, tributyl tin benzoate, parabens, 2,5-dimethyl-N-cyclohexyl-N-methoxy-3-furan carboxamide, 5-ethoxy-3-trichloromethyl-1,2,4 thiadiazole, 3-(2-methyl piperidino) propyl 3,4-dichlorobenzoate, N,N'-(1,4-piperazinediyl bis(2,2,2-trichloro) ethylidene) bis formamide, tetramethylthiuram disulphide, O-Ethyl-S,S-diphenyl-dithiophosphate, 5,10-dihydro-5,10-dioxonaphtho(2,3,9)-p-dithiin-2,3-dicarbonitrile, α-2-[(4-chlorophenyl)ethyl]-α-(1,1-dimethyl ethyl)-1H-1,2,4-triazole-1-ethanol, 3-(3,4-dichlorophenyl)-1,1-dimethylurea, N-tridecyl-2,6-dimethyl-morpholine and 4-N-dodecyl-2,6-dimethylmorpholine, chloramphenicol, alexidine, poly(hexamethylenebicyanoguanide-hexamethylenediamine) hydrochloride, didecyldimethylammonium chloride, 4,4'-diaminodiphenylsulfone, 4-chloro-3-methylphenol, 4-chloro-2-methylphenol, and mixtures of the foregoing.

More preferably, the active ingredient is selected from L-carvone, chloramphenicol, curcumin, 2-phenylphenol, vanillin and mixtures thereof. Most preferably, the active compound according to the present invention is chloramphenicol or 2-phenylphenol.

The inventors surprisingly found that surface-reacted calcium carbonate may be used for preparing aqueous systems comprising said active ingredient in dissolved form, wherein the mass concentration of dissolved active ingredient in said aqueous system corresponds to a supersaturated state. The active ingredients according to the present invention have a solubility limit in water of less than 10 g/l, measured at 20 °C and 1 bar.

Supersaturated aqueous systems comprising dissolved active ingredients which have a very poor solubility in water would be particularly desirable as under conventional conditions, these active ingredients show a very limited bioavailability. High dosages and/or frequent applications are usually required in order to achieve the desired efficacy. However, it was surprisingly found that the supersaturated systems of the present invention may also be prepared with active ingredients having a solubility limit in water of far below 10 g/l, measured at 20 °C and 1 bar. In a preferred embodiment, the active ingredient thus has a solubility limit in water, measured at 20 °C and 1 bar, of less than 5 g/l, preferably less than 3 g/l, more preferably less than 1 g/l, and most preferably less than 0.1 g/l.

### (C) Preparation of the loaded particulate carrier

According to the present invention, surface-reacted calcium carbonate is used as a carrier which is loaded with a poorly water-soluble active ingredient for preparing an aqueous system, wherein the mass concentration of dissolved active ingredient in said aqueous system corresponds to a supersaturated state.

The loading of the surface-reacted calcium carbonate with the active ingredient is effected by contacting the particulate carrier with said active ingredient to form a loaded particulate carrier. A typical process for the preparation of a loaded particulate carrier in the meaning of the present invention comprises at least the following steps:
(a) providing surface-reacted calcium carbonate;
(b) providing an active ingredient having a solubility limit in water of less than 10 g/l, measured at 20 °C and 1 bar;
(c) loading the surface-reacted calcium carbonate provided in step (a) with the active ingredient provided in step (b) to obtain a loaded particulate carrier.

In view of the above, one aspect of the present invention relates to the use of a loaded particulate carrier comprising surface-reacted calcium carbonate loaded with an active ingredient, said active ingredient having a solubility limit in water of less than 10 g/l, measured at 20 °C and 1 bar,
characterized in that the loaded particulate carrier is used for preparing an aqueous system comprising said active ingredient in dissolved form, wherein the mass concentration of dissolved active ingredient in said aqueous system corresponds to a supersaturated state and wherein the loaded particulate carrier is obtainable by a process comprising at least the following steps:
(a) providing surface-reacted calcium carbonate;
(b) providing an active ingredient having a solubility limit in water of less than 10 g/l, measured at 20 °C and 1 bar;
(c) loading the surface-reacted calcium carbonate provided in step (a) with the active ingredient provided in step (b) to obtain a loaded particulate carrier.

Details regarding the surface-reacted calcium carbonate serving as particulate carrier provided in step (a) and the active ingredient provided in step (b) have already been disclosed in the previous sections and shall apply accordingly.

For the purpose of loading step (c), the surface-reacted calcium carbonate may be provided in dry form (i.e. as a powder) or in the form of a suspension in a suitable liquid medium, for example in the form of an aqueous suspension or a slurry. In a similar manner, the active ingredient may be provided in neat form, in the form of a solution or suspension in a suitable liquid medium, or in the form of a melt.

The loading step (c) may be carried out by means of one or more of the following methods:
(i) dry impregnation, i.e. loading a dry particulate carrier with an active ingredient which is in neat form or in the form of a suspension or solution, preferably in a mixing device;
(ii) wet impregnation, i.e. loading the particulate carrier being in the form of a suspension with the active ingredient, preferably in a mixing device;
(iii) melt impregnation, i.e. loading the dry particulate carrier with a melt of the active ingredient in a heated mixer (e.g. a fluid bed mixer).
Dry impregnation, sometimes referred to as incipient wetness impregnation (IWI) or capillary impregnation, is a commonly used technique to load high surface area solid particulate materials with an active ingredient.

Preferably, step (c) is thus carried out by means of dry impregnation. More preferably, the active ingredient provided in step (b) being in the form of a suspension or solution is added dropwise to the surface-reacted calcium carbonate provided in step (a) being in dry form.

One further aspect of the present invention thus relates to the use of a loaded particulate carrier comprising surface-reacted calcium carbonate loaded with an active ingredient, said active ingredient having a solubility limit in water of less than 10 g/l, measured at 20 °C and 1 bar,

characterized in that the loaded particulate carrier is used for preparing an aqueous system comprising said active ingredient in dissolved form, wherein the mass concentration of dissolved active ingredient in said aqueous system corresponds to a supersaturated state and the loaded particulate carrier is obtainable by a process comprising at least the following steps:
(a) providing surface-reacted calcium carbonate;
(b) providing an active ingredient having a solubility limit in water of less than 10 g/l, measured at 20 °C and 1 bar;
(c) loading the surface-reacted calcium carbonate provided in step (a) with the active ingredient provided in step (b) to obtain a loaded particulate carrier;
and wherein loading step (c) is carried out by means of dry impregnation.

In a particularly preferred embodiment, step (c) is carried out by means of dry impregnation, wherein the active ingredient is provided in the form of a solution in an organic solvent, preferably selected from toluene, acetone and ethanol, which is added dropwise to the surface-reacted calcium carbonate provided in step (a) being in dry form.

A "dry" material (e.g. dry surface-reacted calcium carbonate) may be defined by its total moisture content which, unless specified otherwise, is less than or equal to 5.0 wt%, preferably less than or equal to 1.0 wt%, more preferably less than or equal to 0.5 wt%, even more preferably less than or equal to 0.2 wt%, and most preferably between 0.03 and 0.07 wt%, based on the total weight of the dried material.

The total moisture content of a dry material may be measured according to the Karl Fischer coulometric titration method, desorbing the moisture in an oven at 220 °C for 10 min and passing it continuously into a Karl Fischer coulometer (Mettler-Toledo coulometric KF Titrator C30, combined with Mettler-Toledo oven DO 0337) using dry nitrogen at 100 ml/min for 10 min. A calibration curve using water should be recorded and a blank of 10 min nitrogen flow without a sample should be taken into account.

The active ingredient may be dissolved in a suitable solvent, preferably selected from toluene, acetone and ethanol. The solution is then added to an amount of dry surface-reacted calcium carbonate having the same pore volume as the volume of the solution that is added. Capillary action draws the solution into the pores of the calcium carbonate carrier. The mixture should be agitated or shaken to facilitate and accelerate liquid distribution. The obtained powder may then be dried to remove the volatile components, preferably under vacuum, depositing the active on the carrier particles inner and outer surface to obtain a loaded particulate carrier.

Hot melt impregnation is a commonly used technique to load meltable compounds onto and into a porous and or high surface area solid particulate material. Typically, the carrier is heated to a temperature above the melting point of the active compound and then blended with a melt of the active compound in a heated suitable device such as an extruder or a ploughshare mixer, kneader or fluid bed mixer. The amount of molten active ingredient should be dosed in an amount below the available intra particle pore volume of the involved porous powder if the powdered form should be maintained.

For the purpose of wet impregnation, the surface-reacted calcium carbonate of step (a) is provided as a suspension or slurry, the suspension or slurry will contain a suitable liquid medium. Preferably, the liquid medium is selected from water, ethanol, ethanol/water mixtures and toluene. Said slurry may have a solids content within the range of from 1 to 90 wt%, preferably from 3 to 60 wt%, more preferably from 5 to 40 wt% and most preferably from 10 to 25 wt%, based on the total weight of the slurry.

In another embodiment, the process for preparing the loaded particulate carrier described hereinabove further comprises a step of drying the loaded particulate carrier, for example obtained in step (c), to provide a dried loaded particulate carrier. In said drying step, volatile components are removed (e.g. residual liquid media or solvents). Removal of volatile components may improve the performance of the loaded particulate carrier as remaining volatile components may lead to a reduction of the amount of dissolved active ingredient in a supersaturated solution.

The surface-reacted calcium carbonate serving as the particulate carrier and the active ingredient may be used in a specific weight ratio. Said weight ratio may influence the release profile of the loaded particulate carrier when introduced into an aqueous environment and also on the concentration of dissolved active ingredient in the resulting supersaturated solution. In one embodiment, the surface-reacted calcium carbonate is used in a weight ratio of from 100:1 to 1:10, preferably 50:1 to 1:2, and most preferably 20:1 to 1:1 on a dry weights basis relative to the weight of the active ingredient.

### (D) The supersaturated solution

According to the present invention an aqueous system, obtainable by contacting water and a loaded particulate carrier comprising surface-reacted calcium carbonate loaded with an active ingredient is provided. Said active ingredient has a solubility limit in water of less than 10 g/l, measured at 20 °C and 1 bar, and the aqueous system comprises said active ingredient in dissolved form, wherein the mass concentration of dissolved active ingredient in said aqueous system corresponds to a supersaturated state.

In order to obtain said supersaturated aqueous system, the loaded particulate carrier (i.e. the surface-reacted calcium carbonate loaded with the active ingredient) and water may be contacted in any conceivable manner.

The preparation of the inventive aqueous system thus comprises the steps of:
(d) providing water;
(e) contacting the loaded particulate carrier (comprising surface-reacted calcium carbonate loaded with the active ingredient) and the water of step (d); and
(f) mixing the loaded particulate carrier and the water contacted in step (e).

Accordingly, another aspect of the present invention relates to a method for preparing an aqueous system comprising an active ingredient in dissolved form, the method comprising the steps of:
(a) providing surface-reacted calcium carbonate;
(b) providing an active ingredient having a solubility limit in water of less than 10 g/l, measured at 20 °C and 1 bar;
(c) loading the surface-reacted calcium carbonate provided in step (a) with the active ingredient provided in step (b) to obtain a loaded particulate carrier;
(d) providing water;
(e) contacting the loaded particulate carrier obtained in step (c) and the water provided in step (d); and
(f) mixing the loaded particulate carrier and the water contacted in step (e);
characterized in that the mass concentration of dissolved active ingredient in said aqueous system corresponds to a supersaturated state, wherein the surface-reacted calcium carbonate is a reaction product of ground natural calcium carbonate (GNCC) or precipitated calcium carbonate (PCC) treated with CO₂ and one or more H₃O⁺ ion donors, wherein the CO₂ is formed in situ by the H₃O⁺ ion donors treatment and/or is supplied from an external source.

The details regarding steps (a) - (c) have already been disclosed in the previous sections.

In general, any type of water may be used in order to prepare the supersaturated aqueous system of the present invention. The water thus may be distilled water, deionized water, tap water or water taken from natural reservoirs or sources (sea water, lake or pool water, ground water, rain water, river water etc.). Preferably, the water is distilled water, deionized water or tap water, and most preferably distilled water or deionized water.

Depending on the type of water, for example provided in step (d), which is used to prepare the inventive aqueous system, the pH value of said system may vary in a wide range, for example between pH 1.5 and 10. Therefore, in one embodiment, the aqueous system has a pH value in the range of from 1.5 to 10, preferably from 3 to 9, more preferably from 4 to 8 and most preferably from 6.5 to 7.5. The pH value of the inventive supersaturated system may be measured, for example, by means of a SevenMulti pH meter from Mettler-Toledo at 25 °C.

The step of contacting water and the surface-reacted calcium carbonate loaded with said active ingredient (i.e. the loaded particulate carrier), for example in method step (e), may be performed by any suitable method known in the art. For example, the surface-reacted calcium carbonate loaded with the active ingredient may be added to the water in one or more portions. For this purpose, the loaded particulate carrier is preferably provided or used as a dried powder, i.e. as a surface-reacted calcium carbonate loaded with said active ingredient in dried form.

Likewise, the step of mixing the loaded particulate carrier with water, for example as described in step (f), may be performed by any conceivable method by using any suitable mixing device known in the art (e.g. shakers, stirrers). In one embodiment, mixing is carried out by means of a shaker and, optionally, under sonication.

The aqueous system of the present invention comprises water serving as a solvent and an active ingredient serving as a solute, wherein said system shows a higher mass concentration of dissolved solute than would exist at equilibrium. The skilled person will thus appreciate that in order to prepare the inventive aqueous systems with a given amount of water (solvent) and under given conditions, a certain amount of active ingredient (solute) is required which, if dissolved completely, should exceed the solubility limit at equilibrium under said given conditions.

Therefore, the loaded particulate carrier of the present invention may be used in an amount such that the theoretical mass concentration of dissolved active ingredient in the supersaturated aqueous solution is at least 2 times, preferably at least 1.5 times, and most preferably at least 1.1 times higher than the solubility limit of said active ingredient under identical conditions. In addition, or alternatively, the loaded particulate carrier (i.e. the surface-reacted calcium carbonate loaded with the active ingredient) is used in an amount such that the theoretical mass concentration of dissolved active ingredient in the aqueous system is at most 10 times, preferably at most 5 times, and most preferably at most 3 times higher than the solubility limit of said active ingredient under identical conditions. The term "theoretical mass concentration" is a calculated value based on the assumption that the amount of added active ingredient is completely dissolved in the given volume of an aqueous system.

The mass concentration of dissolved active ingredient in the inventive aqueous system may depend on the amount of loaded particulate carrier used and, thus, may also depend on the theoretical mass concentration of the active ingredient. In some embodiments of the present invention, the mass concentration of dissolved active ingredient in the aqueous system is at least 1.1 times, preferably at least 1.5 times, and most preferably at least 2 times higher than the solubility limit of said active ingredient under identical conditions. In a further embodiment, the mass concentration of dissolved active ingredient in the supersaturated aqueous solution may be at least 2.5 times and preferably at least 3 times higher than the solubility limit of said active ingredient under identical conditions.

Since the aqueous system of the present invention is obtainable by contacting water and a loaded particulate carrier, further dissolved or undissolved components (e.g. surface-reacted calcium carbonate) may be present in said system. In one embodiment, the aqueous system according to the present invention thus further comprises a surface-reacted calcium carbonate.

The method for preparing the inventive aqueous system may further comprise an optional separation step following mixing step (f) in order to remove undissolved matter such as carrier particles or undissolved excess active ingredient. In a preferred embodiment, said separation step is carried out by means of centrifugation or filtration. Centrifugation may be preferred to avoid the precipitation of dissolved active ingredient present in the supersaturated aqueous system.

The details of the present invention may be better understood based on the following exemplary embodiment: A solution of a poorly water-soluble active ingredient in ethanol is added dropwise under continuous shaking to dry surface-reacted calcium carbonate to obtain a loaded particulate carrier. In this exemplary embodiment, the particulate carrier is used in a weight ratio of approx. 17:1 on a dry weights basis relative to the weight of the active ingredient. After completion of the addition, residual solvent is optionally removed under reduced pressure to yield a dried surface-reacted calcium carbonate loaded with an active ingredient, i.e. a dried loaded particulate carrier. In the present exemplary embodiment, the active ingredient has a solubility limit in water of 0.6 g/l, measured at 20 °C and 1 bar. For the purpose of preparing the inventive aqueous system, the loaded particulate carrier is added to 1 1 of deionized water (20 °C, 1 bar ambient pressure) under stirring which is continued for 10 min before allowing the system to settle. The amount of loaded particulate carrier is such that the theoretical mass concentration of active ingredient in the aqueous system is 8 times higher than the solubility limit of said active ingredient under identical conditions (0.6 g/l at 20 °C and 1 bar, deionized water), meaning that the added amount of loaded particulate carrier is such that the amount of added active ingredient is 4.8 g/l. After settling the system for 30 min, the concentration of dissolved active ingredient present in the supernatant solution is determined using UV-VIS spectroscopy and a calibration curve obtained with standard solutions of the active ingredient in deionized water. The measurement is carried out at 20 °C and 1 bar ambient pressure. The mass concentration of dissolved active ingredient is 1.8 g/l while, under identical conditions (i.e. 20 °C, 1 bar, deionized water), the solubility limit would be 0.6 g/l. The mass concentration of dissolved active ingredient in the aqueous system is thus 3 times higher than the solubility limit of the active ingredient under identical conditions.

In general, the preparation of supersaturated solvent systems requires the application of very specific conditions in terms of temperature and pressure and leads to the formation of unstable systems which are difficult to handle. By contrast, the use of surface-reacted calcium carbonate as a carrier for poorly water-soluble active ingredients allows for the preparation of supersaturated solutions of said active ingredients by simple addition or mixing.

The inventive system provides an increased effective amount of said poorly water-soluble active ingredients compared with the active ingredient alone or the active ingredient present in conventional formulations.

The inventive system further provides a higher concentration of dissolved active ingredient and thus may be applied less frequently and/or at lower overall dosages without affecting the overall performance of the application.

As was surprisingly found by the inventors, the concentration of dissolved active ingredient in the aqueous system, obtainable by contacting water and the loaded particulate carrier, may remain at a constant level for a period of some minutes up to several hours. The inventive aqueous system may thus be stored, transported and applied without a significant loss of efficacy or without loss of available active ingredient. Because of the higher concentration, overall costs for packaging, storage and shipping of dissolved active ingredients may be reduced.

If the active ingredient is an agrochemical compound, such as a pesticide or a fertilizer, which must be applied by means of a spraying device, the higher concentration of dissolved material also will allow for a faster and less costly application.

In view of the above, one further aspect of the present invention relates to the use of the inventive aqueous system, obtainable by contacting water and the loaded carrier, in agricultural applications.

One further aspect relates to the inventive aqueous system for use as a medicament, for example in the treatment of human and/or animal diseases.

### Description of the figures:

- Fig. 1:: Supersaturation observed with surface-reacted calcium carbonate (SRCC) loaded with chloramphenicol (CAM)
- Fig. 2:: Supersaturation observed with surface-reacted calcium carbonate (SRCC) loaded with chloramphenicol (CAM)
- Fig. 3:: Supersaturation observed with surface-reacted calcium carbonate (SRCC) loaded with 2-phenylphenol (OPP)

### Examples

The scope and interest of the invention may be better understood on basis of the following examples which are intended to illustrate embodiments of the present invention.

### (A) Analytical methods

All parameters defined throughout the present application and mentioned in the following examples are based on the following measuring methods:

### Particle size distributions

The particle size of surface-reacted calcium carbonate herein is described as volume-based particle size distribution *dₓ*(vol). The volume-based median particle size *d*₅₀(vol) and the volume-based top cut particle size *d*₉₈(vol) were evaluated using a Malvern Mastersizer 2000 Laser Diffraction System (Malvern Instruments Plc., Great Britain). The raw data obtained by the measurement was analyzed using the Mie theory, with a particle refractive index of 1.57 and an absorption index of 0.005. The methods and instruments are known to the skilled person and are commonly used to determine particle size distributions of fillers and pigments.

The particle size of particulate materials other than surface-reacted calcium carbonate is described herein as weight-based particle size distribution *dₓ*(wt). The weight determined median particle size *d*₅₀(wt) and top cut *d*₉₈(wt) were measured by the sedimentation method, which is an analysis of sedimentation behaviour in a gravimetric field. The measurement was made with a Sedigraph^{™} 5120 of Micromeritics Instrument Corporation, USA. The method and the instrument are known to the skilled person and are commonly used to determine particle size distributions of fillers and pigments. The measurement was carried out in an aqueous solution of 0.1 wt% Na₄P₂O₇. The samples were dispersed using a high speed stirrer and sonicated.

### BET specific surface area (SSA)

Throughout the present document, the specific surface area (in m²/g) was determined using the BET method (using nitrogen as adsorbing gas), which is well known to the skilled man (ISO 9277:2010). The total surface area (in m²) of the filler material was then obtained by multiplication of the specific surface area and the mass (in g) of the corresponding sample.

### Porosimetry

The specific pore volume is measured using a mercury intrusion porosimetry measurement using a Micromeritics Autopore V 9620 mercury porosimeter having a maximum applied pressure of mercury 414 MPa (60 000 psi), equivalent to a Laplace throat diameter of 0.004 µm. The equilibration time used at each pressure step is 20 s. The sample material is sealed in a 3 cm³ chamber powder penetrometer for analysis. The data are corrected for mercury compression, penetrometer expansion and sample material elastic compression using the software Pore-Comp (Gane, P.A.C., Kettle, J.P., Matthews, G.P. and Ridgway, C.J., "Void Space Structure of Compressible Polymer Spheres and Consolidated Calcium Carbonate Paper-Coating Formulations", Industrial and Engineering Chemistry Research, 1996, 35(5), 1753 - 1764).

The total pore volume seen in the cumulative intrusion data can be separated into two regions with the intrusion data from 214 µm down to about 1 to 4 µm showing the coarse packing of the sample between any agglomerate structures contributing strongly. Below these diameters lies the fine interparticle packing of the particles themselves. If they also have intraparticle pores, then this region appears bimodal, and by taking the specific pore volume intruded by mercury into pores finer than the modal turning point, i.e. finer than the bimodal point of inflection, we thus define the specific intraparticle pore volume. The sum of these three regions gives the total overall pore volume of the powder, but depends strongly on the original sample compaction/settling of the powder at the coarse pore end of the distribution.

By taking the first derivative of the cumulative intrusion curve, the pore size distributions based on equivalent Laplace diameter, inevitably including pore-shielding, are revealed. The differential curves clearly show the coarse agglomerate pore structure region, the interparticle pore region and the intraparticle pore region, if present. Knowing the intraparticle pore diameter range it is possible to subtract the remainder interparticle and interagglomerate pore volume from the total pore volume to deliver the desired pore volume of the internal pores alone in terms of the pore volume per unit mass (specific pore volume). The same principle of subtraction, of course, applies for isolating any of the other pore size regions of interest.

### Mass concentration

The mass concentration of a solute (e.g. dissolved active ingredient) present in a solution or solvent system can be determined according to methods generally known to the skilled person. Suitable methods according to the present invention include UV-VIS spectroscopy, chromatographic methods as well as gravimetric or volumetric methods. Preferably, UV-VIS spectroscopy may be used to determine the mass concentration.

### Thermogravimetric analysis (TGA)

Thermogravimetric analysis was performed on a Mettler-Toledo TGA/DSC1 (TGA 1 STARe System) instrument with a method as follows: 30 - 80 °C and hold 5 min (10 K/min), 80 - 110 °C and hold 5 min (10 K/min), 10 - 570 °C and hold 10 min (20 K/min).

### Solubility limit

The solubility limit is determined by the shake flask method known to the skilled person. According to this method, excess compound (e.g. active ingredient) is added to the solvent (e.g. water, preferably deionized water) and shaken at 20 °C and 1 bar ambient pressure for at least 24 h. The saturation is confirmed by observation of the presence of undissolved material. After filtration of the slurry, a sample of the solution for analysis is taken. Both filtration and analysis is performed under the conditions used during dissolution (20 °C, 1 bar) to minimize loss of volatile components. If necessary, the sample may be diluted to prevent crystallization. The mass concentration of solute contained in the sample is then determined by an appropriate known method which depends on the nature of the solute/solvent and on the concentration.

In many cases, solubility limits of active ingredients are available in public databases, for example *GESTIS Gefahrstoffdatenbank.* In case of any differences or inconsistencies, the solubility limit determined according to the method described hereinabove shall be preferred.

### (B) Examples

The following examples are not to be construed to limit the scope of the claims in any manner whatsoever.

### Example 1: Preparation of surface-reacted calcium carbonate

In a mixing vessel, 330 1 of an aqueous suspension of calcium carbonate-containing mineral was prepared by adjusting the solids content of a ground limestone calcium carbonate from Omya SAS, Orgon, having a weight based median particle size of 1.3 µm, as determined by sedimentation, such that a solids content of 10 wt%, based on the total weight of the aqueous suspension, was obtained.

Whilst mixing the suspension at a mixer tip speed of 12.7 m/s, 10.6 kg of an aqueous solution containing 30 wt% phosphoric acid, based on the total weight of the aqueous solution, was added to said suspension over a period of 12 min at a temperature of 70 °C. After the addition of the acid, the slurry was stirred for additional 5 min, before removing it from the vessel and drying. During acid treatment, carbon dioxide was formed in situ in the aqueous suspension.

The resulting surface-reacted calcium carbonate SRCC1 had an intraparticle intruded specific pore volume of 0.871 g/cm³ for the pore diameter range of 0.004 to 0.4 µm (using a Micromeritics Autopore IV 9500 mercury porosimeter having a maximum applied pressure of 414 MPa with a equilibration time used at each pressure step of 20 seconds; the sample material was sealed in a 5 ml chamber powder penetrometer for analysis), a volume median grain diameter (*d*₅₀) of 7.3 µm and a *d*₉₈ of 16.6 µm as measured by laser diffraction (Malvern Mastersizer 2 000) and a specific surface area of 52.1 m²/g.

### Example 2: Supersaturated solution of chloramphenicol

### Loading:

1.100 g of surface-reacted calcium carbonate prepared as described above (*d*₅₀(vol) ≈ 7 µm, *d*₉₈(vol) ≈ 16 µm, SSA ≈ 55 m²/g) were shaken in a 1 l Erlenmeyer flask using a Heidolph Uni 2010 orbital shaker at 300 rpm. A stock solution of 133 g/ml chloramphenicol (dry, purchased from Merck, #1.02366.0050) in absolute ethanol was added dropwise at a speed of 1.5 drops/second using a burette until the SRCC powder was loaded with 8.65 % w/w of chloramphenicol. The sample was dried at room temperature. The percentage of loading and absence of ethanol was confirmed by TGA.

### Calibration curve:

The following standard solutions of chloramphenicol in water were prepared and analyzed by UV-VIS: 0.013 mg/ml, 0.083 mg/ml, 0.113 mg/ml, 0.167 mg/ml, and 0.333 mg/ml. The absorbance of each 3 µl of standard was measured at 280 nm in a Nanodrop 2000c device and was taken as a reference to determine a calibration curve "mg/ml chloramphenicol vs. absorbance (280 nm)". A linear fit analysis was performed (*R*² = 0.9982).

### Solubility measurements:

1. The SRCC loaded with chloramphenicol as prepared above was mixed with water at a theoretical mass concentration of 5 mg/ml which is higher than the solubility limit reported in the product datasheet (2.5 mg/ml). The identical amount of pure chloramphenicol was taken as a reference.
2. After shaking, 0.1 ml of the solution were removed and centrifuged at 16 000 rcf (relative centrifugal force) for 1 min. The supernatant was then diluted with water at a ratio of 1:20 (5 µl + 95 µl), mixed by pipetting five times (95 µl volume) and absorbency was then measured immediately as described above.
3. The linear fit analysis was used to calculate the concentration of dissolved chloramphenicol in water.
4. Steps 2 and 3 were repeated at different time points.

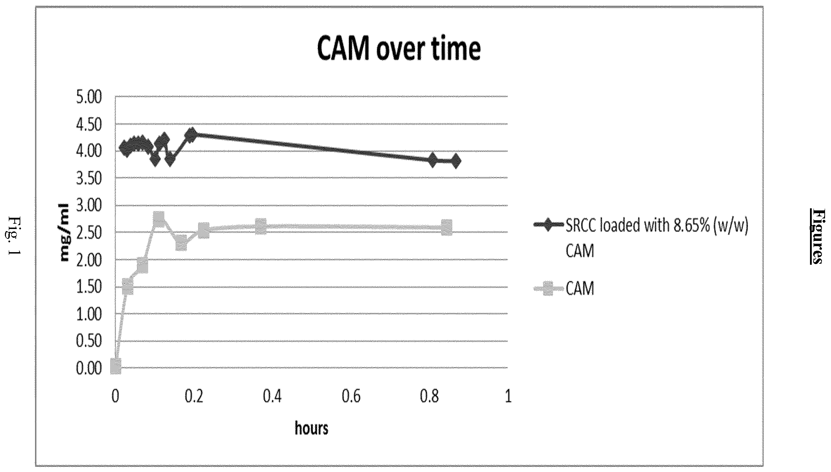

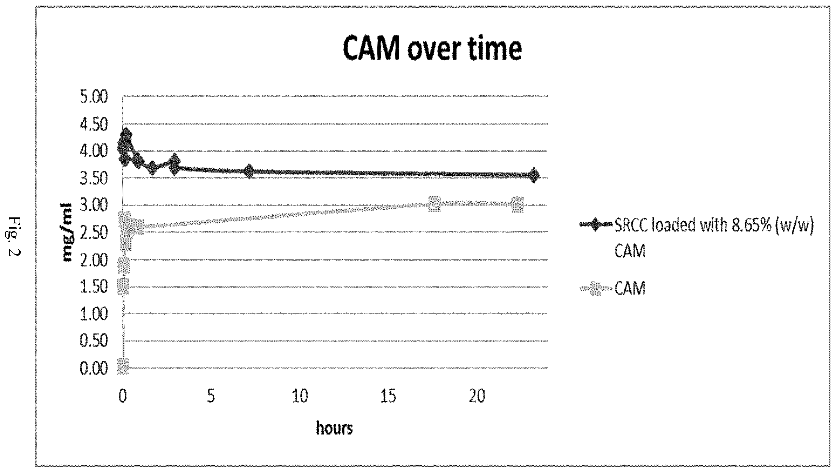

The results of the concentration measurements are shown in Fig. 1 and Fig. 2 and clearly indicate the presence of a supersaturated aqueous system as the concentration of active ingredient (chloramphenicol) dissolved in the supernatant solution exceeds the reported solubility limit (2.5 mg/ml).

### Example 3: Supersaturated solution of 2-phenylphenol

### Loading:

50 g of surface-reacted calcium carbonate prepared as described above (*d*₅₀(vol) ≈ 7 µm, *d*₉₈(vol) ≈ 16 µm, SSA ≈ 55 m²/g) were shaken in a 1 l Erlenmeyer flask using a Heidolph Uni 2010 orbital shaker at 300 rpm. A stock solution of 75 % w/w 2-phenylphenol in absolute ethanol (purchased from Lanxess, Preventol O Extra) was added dropwise at a speed of 1.5 drops/s using a burette until the SRCC powder was loaded with 17.2 % w/w of 2-phenylphenol. The sample was dried at room temperature. The percentage of loading and absence of ethanol was confirmed by TGA.

### Calibration curve:

The following standard solutions of 2-phenylphenol in water were prepared and analyzed by UV-VIS: 0.025 mg/ml, 0.05 mg/ml, 0.1 mg/ml, 0.2 mg/ml, 0.4 mg/ml, 0.6 mg/ml and 0.8 mg/ml. The absorbance of each 3 µl of standard was measured at 283 nm in a Nanodrop 2000c device and was taken as a reference to determine a calibration curve "mg/ml 2-phenylphenol vs. absorbance (283 nm)". A linear fit analysis was performed (*R*² = 0.9996).

### Solubility measurements:

1. The SRCC loaded with 2-phenylphenol as prepared above was mixed with water at a theoretical mass concentration of 4.8 mg/ml which is higher than the solubility limit reported in the product datasheet (0.5 to 0.6 mg/ml). The identical amount of pure 2-phenylphenol was taken as a reference.
2. After shaking, 0.1 ml of the solution were removed and centrifuged at 16 000 rcf for 1 minute. A dilution of 1:10 in water of the supernatant was performed to measure absorbency. Absorbency was then measured immediately as described above.
3. The linear fit analysis was used to calculate the concentration of dissolved 2-phenylphenol in water.
4. Steps 2 and 3 were repeated at different time points.

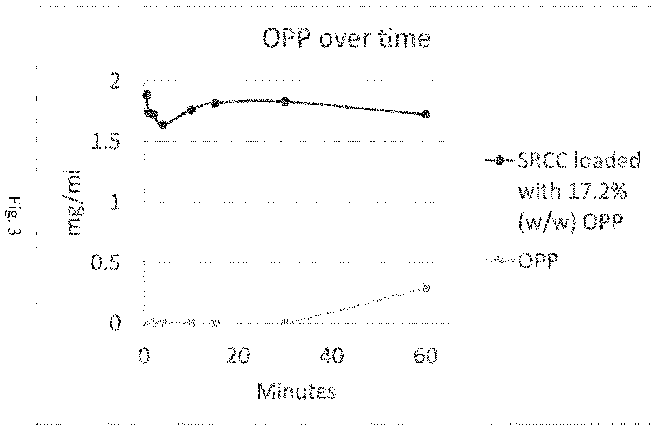

The results of the concentration measurements are shown in Fig. 3 and clearly indicate the presence of a supersaturated system as the concentration of active ingredient (2-phenylphenol) dissolved in the supernatant solution exceeds the reported solubility limit (0.5 to 0.6 mg/ml).

## Claims

1. Use of a loaded particulate carrier comprising surface-reacted calcium carbonate loaded with an active ingredient, said active ingredient having a solubility limit in water of less than 10 g/l, measured at 20 °C and 1 bar,
**characterized in that** the loaded particulate carrier is used for preparing an aqueous system comprising said active ingredient in dissolved form, wherein the mass concentration of dissolved active ingredient in said aqueous system corresponds to a supersaturated state, wherein the surface-reacted calcium carbonate is a reaction product of ground natural calcium carbonate (GNCC) or precipitated calcium carbonate (PCC) treated with CO₂ and one or more H₃O⁺ ion donors, wherein the CO₂ is formed in situ by the H₃O⁺ ion donors treatment and/or is supplied from an external source.

2. The use according to claim 1, **characterized in that** the one or more H₃O⁺ ion donor is selected from a strong acid having a pKₐ of 0 or less, medium-strong acid having a pKₐ in the range from 0 to 2.5, weak acid having a pKₐ value of greater than 2.5 and less than or equal to 7, when measured at 20 °C, associated with the ionisation of the first available hydrogen, or acidic salts thereof or mixtures thereof.

3. The use according to claim 1, **characterized in that** the surface-reacted calcium carbonate is obtained by a process comprising the steps of:
(a) providing a ground natural calcium carbonate (GNCC) or precipitated calcium carbonate (PCC);
(b) providing at least one acid;
(c) providing gaseous CO₂; and
(d) contacting said GNCC or PCC provided in step (a), the at least one acid provided in step (b) and the gaseous CO₂ provided in step (c);
**characterized in that**
(i) the at least one acid provided in step (b) has a pKₐ of greater than 2.5 and less than or equal to 7 at 20 °C, associated with the ionisation of its first available hydrogen, and a corresponding anion is formed on loss of this first available hydrogen capable of forming a water-soluble calcium salt; and
(ii) following contacting the at least one water-soluble acid provided in step (b) and the GNCC or PCC provided in step (a), at least one water-soluble salt, which in the case of a hydrogen-containing salt has a pKₐ of greater than 7 at 20 °C, associated with the ionisation of the first available hydrogen, and the salt anion of which is capable of forming water-insoluble calcium salts, is additionally provided.

4. The use according to any of claims 1 to 3, **characterized in that** the surface-reacted calcium carbonate has:
(i) a specific surface area of from 15 m²/g to 200 m²/g, preferably from 27 m²/g to 180 m²/g, more preferably from 30 m²/g to 160 m²/g, even more preferably from 45 m²/g to 150 m²/g, most preferably from 48 m²/g to 140 m²/g, measured using nitrogen and the BET method. measured using nitrogen and the BET method according to ISO 9277:2010;
(ii) a volume median grain diameter dso(vol) of from 1 to 75 µm, preferably from 2 to 50 µm, more preferably 3 to 40 µm, even more preferably from 4 to 30 µm, and most preferably from 5 to 15 µm;
(iii) a grain diameter *d*₉₈(vol) of from 2 to 150 µm, preferably from 4 to 100 µm, more preferably 6 to 80 µm, even more preferably from 8 to 60 µm, and most preferably from 10 to 30 µm; and/or
(iv) an intra-particle intruded specific pore volume in the range from 0.1 to 2.3 cm³/g, more preferably from 0.2 to 2.0 cm³/g, especially preferably from 0.4 to 1.8 cm³/g and most preferably from 0.6 to 1.6 cm³/g, calculated from mercury porosimetry measurement.

5. The use according to any of claims 1 to 4, **characterized in that** the active ingredient has a solubility limit in water, measured at 20 °C and 1 bar, of less than 5 g/l, preferably less than 1 g/l, and most preferably less than 0.1 g/l.

6. The use according to any of claims 1 to 5, **characterized in that** the surface-reacted calcium carbonate is used in a weight ratio of from 100:1 to 1:10, preferably 50:1 to 1:2, and most preferably 20:1 to 1:1 on a dry weights basis relative to the weight of the active ingredient.

7. The use according to any of claims 1 to 6, **characterized in that** the loaded particulate carrier is used in an amount such that the theoretical mass concentration of dissolved active ingredient in the aqueous system is at most 10 times, preferably at most 5 times, and most preferably at most 3 times higher than the solubility limit of said active ingredient under identical conditions.

8. The use according to any of claims 1 to 7, **characterized in that** the mass concentration of dissolved active ingredient in the aqueous system is at least 1.1 times, preferably at least 1.5 times, and most preferably at least 2 times higher than the solubility limit of said active ingredient under identical conditions.

9. The use according to any of claims 1 to 8, **characterized in that** the aqueous system has a pH value in the range of from 1.5 to 10, preferably from 3 to 9, more preferably from 4 to 8 and most preferably from 6.5 to 7.5.

10. The use according to any of claims 1 to 9, **characterized in that** the active ingredient is selected from pharmaceutical drugs, agrochemical compounds including pesticides and fertilizers, biocides, micronutrients, antimicrobial agents including antifungal agents and antibacterial agents, and mixtures thereof; preferably the biocide is selected from the group consisting of phenols, halogenated phenols, halogen-containing compounds, halogen-releasing compounds, isothiazolinones, aldehyde-containing compounds, aldehyde-releasing compounds, biguanides, sulfones, thiocyanates, pyrithiones, antibiotics such as β-lactam antibiotics,
quaternary ammonium salts, peroxides, perchlorates, amides, amines, heavy metals, biocidal enzymes, biocidal polypeptides, azoles, carbamates, glyphosates, sulphonamides and mixtures thereof; more preferably the active ingredient is selected from L-carvone, chloramphenicol, curcumin, 2-phenylphenol, vanillin and mixtures thereof; most preferably the active compound is chloramphenicol or 2-phenylphenol.

11. An aqueous system, obtainable by contacting water and a loaded particulate carrier comprising surface-reacted calcium carbonate loaded with an active ingredient, said active ingredient having a solubility limit in water of less than 10 g/l, measured at 20 °C and 1 bar,
**characterized in that** the aqueous system comprises said active ingredient in dissolved form, wherein the mass concentration of dissolved active ingredient in said aqueous system corresponds to a supersaturated state wherein the surface-reacted calcium carbonate is a reaction product of ground natural calcium carbonate (GNCC) or precipitated calcium carbonate (PCC) treated with CO₂ and one or more H₃O⁺ ion donors, wherein the CO₂ is formed in situ by the H₃O⁺ ion donors treatment and/or is supplied from an external source..

12. A method for preparing an aqueous system comprising an active ingredient in dissolved form, the method comprising the steps of:
(a) providing surface-reacted calcium carbonate;
(b) providing an active ingredient having a solubility limit in water of less than 10 g/l, measured at 20 °C and 1 bar;
(c) loading the surface-reacted calcium carbonate provided in step (a) with the active ingredient provided in step (b) to obtain a loaded particulate carrier;
(d) providing water;
(e) contacting the loaded particulate carrier obtained in step (c) and the water provided in step (d); and
(f) mixing the loaded particulate carrier and the water contacted in step (e);
**characterized in that** the mass concentration of dissolved active ingredient in said aqueous system corresponds to a supersaturated state, wherein the surface-reacted calcium carbonate is a reaction product of ground natural calcium carbonate (GNCC) or precipitated calcium carbonate (PCC) treated with CO₂ and one or more H₃O⁺ ion donors, wherein the CO₂ is formed in situ by the H₃O⁺ ion donors treatment and/or is supplied from an external source.

## Patentansprüche

1. Verwendung eines beladenen teilchenförmigen Trägers, umfassend oberflächenreagiertes Calciumcarbonat, das mit einem Wirkstoff beladen ist, wobei der Wirkstoff einen Löslichkeitsgrenzwert in Wasser von kleiner als 10 g/l, gemessen bei 20 °C und 1 Bar, aufweist,
**dadurch gekennzeichnet, dass** der beladene teilchenförmige Träger zum Zubereiten eines wässrigen Systems verwendet wird, das den Wirkstoff in aufgelöster Form umfasst, wobei die Massenkonzentration an gelöstem Wirkstoff in dem wässrigen System einem supergesättigten Zustand entspricht, wobei das oberflächenreagierte Calciumcarbonat ein Reaktionsprodukt von gemahlenem natürlichen Calciumcarbonat (GNCC) oder ausgefälltem Calciumcarbonat (PCC) ist, das mit CO₂ und einem oder mehreren H₃O⁺-Ionendonatoren behandelt ist, wobei das CO₂ in situ durch die H₃O⁺-Ionendonatorenbehandlung gebildet wird und/oder von einer externen Quelle zugeführt wird.

2. Verwendung nach Anspruch 1, **dadurch gekennzeichnet, dass** der eine oder die mehreren H₃O⁺-Ionendonatoren aus einer starken Säure mit einem pKₐ von 0 oder kleiner, einer mittelstarken Säure mit einem pKₐ im Bereich von 0 bis 2,5, einer schwachen Säure mit einem pKₐ-Wert von größer 2,5 und kleiner oder gleich 7, wenn bei 20 °C gemessen, in Verbindung mit der Ionisierung des ersten verfügbaren Wasserstoffs, oder sauren Salzen davon oder Gemischen davon ausgewählt sind.

3. Verwendung nach Anspruch 1, **dadurch gekennzeichnet, dass** das oberflächenreagierte Calciumcarbonat durch einen Prozess erhalten wird, der die folgenden Schritte umfasst:
(a) Bereitstellen eines gemahlenen natürlichen Calciumcarbonats (GNCC) oder ausgefällten Calciumcarbonats (PCC);
(b) Bereitstellen mindestens einer Säure;
(c) Bereitstellen von gasförmigen CO₂; und
(d) In-Kontakt-Bringen des GNCC oder PCC, das in Schritt (a) bereitgestellt wurde, der mindestens einen Säure, die in Schritt (b) bereitgestellt wurde, und des gasförmigen CO₂, das in Schritt (c) bereitgestellt wurde;
**dadurch gekennzeichnet, dass**
(i) die mindestens eine Säure, die in Schritt (b) bereitgestellt wurde, einen pKₐ größer als 2,5 und kleiner oder gleich 7 bei 20 °C, verbunden mit der Ionisierung ihres ersten verfügbaren Wasserstoffs, aufweist und ein entsprechendes Anion bei Verlust dieses ersten verfügbaren Wasserstoffs gebildet wird, das imstande ist, ein wasserlösliches Calciumsalz zu bilden; und
(ii) nach In-Kontakt-Bringen der mindestens einen wasserlöslichen Säure, die in Schritt (b) bereitgestellt wurde, und des GNCC oder PCC, das in Schritt (a) bereitgestellt wurde, zusätzlich mindestens ein wasserlösliches Salz bereitgestellt ist, das im Fall eines Wasserstoff-haltigen Salzes einen pKₐ von größer 7 bei 20 °C, verbunden mit der Ionisierung des ersten verfügbaren Wasserstoffs aufweist und dessen Salzanion imstande ist, wasserunlösliche Calciumsalze zu bilden.

4. Verwendung nach einem der Ansprüche 1 bis 3, **dadurch gekennzeichnet, dass** das oberflächenreagierte Calciumcarbonat Folgendes aufweist:
(i) eine spezifische Oberfläche von 15 m²/g bis 200 m²/g, bevorzugt von 27 m²/g bis 180 m²/g, bevorzugter von 30 m²/g bis 160 m²/g, noch bevorzugter von 45 m²/g bis 150 m²/g, am bevorzugtesten von 48 m²/g bis 140 m²/g, gemessen unter Verwendung von Stickstoff und dem BET-Verfahren, gemessen unter Verwendung von Stickstoff und dem BET-Verfahren gemäß ISO 9277:2010;
(ii) einen mittleren Kornvolumendurchmesser d₅₀(vol) von 1 bis 75 µm, bevorzugt von 2 bis 50 µm, bevorzugter 3 bis 40 µm, noch bevorzugter von 4 bis 30 µm und am bevorzugtesten von 5 bis 15 µm;
(iii) einen Korndurchmesser d₉₈(vol) von 2 bis 150 µm, bevorzugt von 4 bis 100 µm, bevorzugter 6 bis 80 µm, noch bevorzugter von 8 bis 60 µm und am bevorzugtesten von 10 bis 30 µm; und/oder
(iv) ein intrapartikuläres, spezifisches Eindringungsporenvolumen im Bereich von 0,1 bis 2,3 cm³/g, bevorzugter von 0,2 bis 2,0 cm³/g, besonders bevorzugt von 0,4 bis 1,8 cm³/g und am bevorzugtesten von 0,6 bis 1,6 cm³/g, berechnet aus Quecksilberporosimetriemessung.

5. Verwendung nach einem der Ansprüche 1 bis 4, **dadurch gekennzeichnet, dass** der Wirkstoff einen Löslichkeitsgrenzwert in Wasser, gemessen bei 20 °C und 1 Bar, von kleiner als 5 g/l, bevorzugt kleiner als 1 g/l, und am bevorzugtesten kleiner als 0,1 g/l aufweist.

6. Verwendung nach einem der Ansprüche 1 bis 5, **dadurch gekennzeichnet, dass** das oberflächenreagierte Calciumcarbonat in einem Gewichtsverhältnis von 100:1 bis 1:10, bevorzugt 50:1 bis 1:2 und am bevorzugtesten 20:1 bis 1:1 auf einer Trockengewichtsbasis relativ zu dem Gewicht des Wirkstoffs verwendet wird.

7. Verwendung nach einem der Ansprüche 1 bis 6, **dadurch gekennzeichnet, dass** der beladene teilchenförmige Träger in einer derartigen Menge verwendet wird, dass die theoretische Massenkonzentration an gelöstem Wirkstoff in dem wässrigen System höchstens 10 Mal, bevorzugt höchstens 5 Mal und am bevorzugtesten höchstens 3 Mal höher ist als der Löslichkeitsgrenzwert des Wirkstoffs unter identischen Bedingungen.

8. Verwendung nach einem der Ansprüche 1 bis 7, **dadurch gekennzeichnet, dass** die Massenkonzentration an gelöstem Wirkstoff in dem wässrigen System mindestens 1,1 Mal, bevorzugt mindestens 1,5 Mal und am bevorzugtesten mindestens 2 Mal höher ist als der Löslichkeitsgrenzwert des Wirkstoffs unter identischen Bedingungen.

9. Verwendung nach einem der Ansprüche 1 bis 8, **dadurch gekennzeichnet, dass** das wässrige System einen pH-Wert im Bereich von 1,5 bis 10, bevorzugt von 3 bis 9, bevorzugter von 4 bis 8 und am bevorzugtesten von 6,5 bis 7,5 aufweist.

10. Verwendung nach einem der Ansprüche 1 bis 9, **dadurch gekennzeichnet, dass** der Wirkstoff ausgewählt ist aus pharmazeutischen Arzneimitteln, agrochemischen Verbindungen, beinhaltend Pestizide und Düngemittel, Bioziden, Mikronährstoffen, antimikrobiellen Mitteln, beinhaltend antifungale Mittel und antibakterielle Mittel, und Gemischen davon; bevorzugt das Biozid ausgewählt ist aus der Gruppe bestehend aus Phenolen, halogenierten Phenolen, halogenhaltigen Verbindungen, halogenfreisetzenden Verbindungen, Isothiazolinonen, aldehydhaltigen Verbindungen, aldehydfreisetzenden Verbindungen, Biguaniden, Sulfonen, Thiocyanaten, Pyrithionen, Antibiotika wie β -Lactam Antibiotika,
quaternären Ammoniumsalzen, Peroxiden, Perchloraten, Amiden, Aminen, Schwermetallen, bioziden Enzymen, bioziden Polypeptiden, Azolen, Carbamaten, Glyphosaten, Sulphonamiden und Gemischen davon; bevorzugter der Wirkstoff ausgewählt ist aus L-Carvon, Chloramphenicol, Curcumin, 2-Phenylphenol, Vanillin und Gemischen davon; am bevorzugtesten die aktive Verbindung Chloramphenicol oder 2-Phenylphenol ist.

11. Wässriges System, das durch In-Kontakt-Bringen von Wasser und einem beladenen teilchenförmigen Träger, umfassend oberflächenreagiertes Calciumcarbonat, das mit einem Wirkstoff beladen ist, erhältlich ist, wobei der Wirkstoff einen Löslichkeitsgrenzwert in Wasser von kleiner als 10 g/l, gemessen bei 20 °C und 1 Bar aufweist,
**dadurch gekennzeichnet, dass** das wässrige System den Wirkstoff in aufgelöster Form umfasst, wobei die Massenkonzentration an gelöstem Wirkstoff in dem wässrigen System einem supergesättigten Zustand entspricht, wobei das oberflächenreagierte Calciumcarbonat ein Reaktionsprodukt von gemahlenem natürlichen Calciumcarbonat (GNCC) oder ausgefälltem Calciumcarbonat (PCC) ist, das mit CO₂ und einem oder mehreren H₃O⁺ Ionendonatoren behandelt ist, wobei das CO₂ in situ durch dieH₃O⁺ Ionendonatorenbehandlung gebildet wird und/oder von einer externen Quelle zugeführt wird.

12. Verfahren zur Herstellung eines wässrigen Systems, das einen Wirkstoff in aufgelöster Form umfasst, wobei das Verfahren die folgenden Schritte umfasst:
(a) Bereitstellen von oberflächenreagiertem Calciumcarbonat;
(b) Bereitstellen eines Wirkstoffs mit einem Löslichkeitsgrenzwert in Wasser von kleiner als 10 g/l, gemessen bei 20 °C und 1 Bar;
(c) Beladen des oberflächenreagierten Calciumcarbonats, das in Schritt (a) bereitgestellt wurde, mit dem Wirkstoff, der in Schritt (b) bereitgestellt wurde, um einen beladenen teilchenförmigen Träger zu erhalten;
(d) Bereitstellen von Wasser;
(e) In-Kontakt-Bringen des beladenen teilchenförmigen Trägers, der in Schritt (c) erhalten wurde, und des Wassers, das in Schritt (d) bereitgestellt wurde; und
(f) Mischen des beladenen teilchenförmigen Trägers und des Wassers, die in Schritt (e) in Kontakt gebracht wurden;
**dadurch gekennzeichnet, dass** die Massenkonzentration an gelöstem Wirkstoff in dem wässrigen System einem supergesättigten Zustand entspricht, wobei das oberflächenreagierte Calciumcarbonat ein Reaktionsprodukt von gemahlenem natürlichen Calciumcarbonat (GNCC) oder ausgefälltem Calciumcarbonat (PCC) ist, das mit CO₂ und einem oder mehreren H₃O⁺-Ionendonatoren behandelt ist, wobei das CO₂ in situ durch die H₃O⁺ Ionendonatorenbehandlung gebildet wird und/oder von einer externen Quelle zugeführt wird.

## Revendications

1. Utilisation d'un support particulaire chargé comprenant du carbonate de calcium ayant réagi en surface chargé d'un ingrédient actif, ledit ingrédient actif présentant une limite de solubilité dans l'eau inférieure à 10 g/l, mesurée à 20 °C et à 1 bar,
**caractérisée en ce que** le support particulaire chargé est utilisé pour préparer un système aqueux comprenant ledit ingrédient actif sous une forme dissoute, dans laquelle la concentration en masse de l'ingrédient actif dissous dans ledit système aqueux correspond à un état sursaturé, dans laquelle le carbonate de calcium ayant réagi en surface est un produit réactionnel de carbonate de calcium naturel (GNCC) ou de carbonate de calcium précipité (PCC) traité avec du CO₂ et un ou plusieurs donneurs d'ions H₃O⁺, dans laquelle le CO₂ est formé in situ par le traitement avec des donneurs d'ions H₃O⁺ et/ou est fourni à partir d'une source externe.

2. Utilisation selon la revendication 1, **caractérisée en ce que** les un ou plusieurs donneurs d'ions H₃O⁺ sont sélectionnés parmi un acide fort présentant un pKₐ de 0 ou moins, un acide moyen-fort présentant un pKₐ dans la plage de 0 à 2,5, un acide faible présentant une valeur pKₐ supérieure à 2,5 et inférieure ou égale à 7, lorsqu'ils sont mesurés à 20 °C, associés à l'ionisation du premier hydrogène disponible, ou des sels acides de ceux-ci ou des mélanges de ceux-ci.

3. Utilisation selon la revendication 1, **caractérisée en ce que** le carbonate de calcium ayant réagi en surface est obtenu par un processus comprenant les étapes consistant à :
(a) fournir un carbonate de calcium naturel (GNCC) ou un carbonate de calcium précipité (PCC) ;
(b) fournir au moins un acide ;
(c) fournir du CO₂ gazeux ; et
(d) mettre en contact ledit GNCC ou PCC fourni à l'étape (a), le au moins un acide fourni à l'étape (b) et le CO₂ gazeux fourni à l'étape (c) ;
**caractérisée en ce que**
(i) le au moins un acide fourni à l'étape (b) présente un pKₐ supérieur à 2,5 et inférieur ou égal à 7 à 20 °C, associé à l'ionisation de son premier hydrogène disponible, et un anion correspondant est formé sur la perte de ce premier hydrogène disponible capable de former un sel de calcium hydrosoluble ; et
(ii) à la suite de l'étape consistant à mettre en contact le au moins un acide hydrosoluble fourni à l'étape (b) et le GNCC ou PCC fourni à l'étape (a), au moins un sel hydrosoluble, qui, dans le cas d'un sel contenant de l'hydrogène, présente un pKₐ supérieur à 7 à 20 °C, associé à l'ionisation du premier hydrogène disponible, et dont l'anion de sel est capable de former des sels de calcium insolubles dans l'eau, est en outre fourni.

4. Utilisation selon l'une quelconque des revendications 1 à 3, **caractérisée en ce que** le carbonate de calcium ayant réagi en surface présente :
(i) une surface spécifique de 15 m²/g à 200 m²/g, de préférence de 27 m²/g à 180 m²/g, plus préférentiellement de 30 m²/g à 160 m²/g, encore plus préférentiellement de 45 m²/g à 150 m²/g, le plus préférentiellement de 48 m²/g à 140 m²/g, mesurée en utilisant de l'azote et le procédé BET, mesurée en utilisant de l'azote et le procédé BET conformément à ISO 9277:2010 ;
(ii) un diamètre de grain moyen en volume d₅₀ (vol) de 1 à 75 µm, de préférence de 2 à 50 µm, plus préférentiellement de 3 à 40 µm, encore plus préférentiellement de 4 à 30 µm, et le plus préférentiellement de 5 à 15 µm ;
(iii) un diamètre de grain d₉₈ (vol) de 2 à 150 µm, de préférence de 4 à 100 µm, plus préférentiellement de 6 à 80 µm, encore plus préférentiellement de 8 à 60 µm, et le plus préférentiellement de 10 à 30 µm ; et/ou
(iv) un volume spécifique des pores imposé intra-particulaire dans la plage de 0,1 à 2,3 cm³/g, plus préférentiellement de 0,2 à 2,0 cm³/g, spécialement de préférence de 0,4 à 1,8 cm³/g et le plus préférentiellement de 0,6 à 1,6 cm³/g, calculé par une mesure de porosimétrie au mercure.

5. Utilisation selon l'une quelconque des revendications 1 à 4, **caractérisée en ce que** l'ingrédient actif présente une limite de solubilité dans l'eau, mesurée à 20 °C et à 1 bar, inférieure à 5 g/l, de préférence inférieure à 1 g/l, et le plus préférentiellement inférieure à 0,1 g/l.

6. Utilisation selon l'une quelconque des revendications 1 à 5, **caractérisée en ce que** le carbonate de calcium ayant réagi en surface est utilisé dans un rapport pondéral de 100:1 à 1:10, de préférence de 50:1 à 1:2, et le plus préférentiellement de 20:1 à 1:1 sur une base de poids secs par rapport au poids de l'ingrédient actif.

7. Utilisation selon l'une quelconque des revendications 1 à 6, **caractérisée en ce que** le support particulaire chargé est utilisé dans une quantité telle que la concentration en masse théorique d'ingrédient actif dissous dans le système aqueux est au plus 10 fois, de préférence au plus 5 fois, et le plus préférentiellement au plus 3 fois supérieure à la limite de solubilité dudit ingrédient actif dans des conditions identiques.

8. Utilisation selon l'une quelconque des revendications 1 à 7, **caractérisée en ce que** la concentration en masse d'ingrédient actif dissous dans le système aqueux est au moins 1,1 fois, de préférence au moins 1,5 fois, et le plus préférentiellement au moins 2 fois supérieure à la limite de solubilité dudit ingrédient actif dans des conditions identiques.

9. Utilisation selon l'une quelconque des revendications 1 à 8, **caractérisée en ce que** le système aqueux présente une valeur pH dans la plage de 1,5 à 10, de préférence de 3 à 9, plus préférentiellement de 4 à 8 et le plus préférentiellement de 6,5 à 7,5.

10. Utilisation selon l'une quelconque des revendications 1 à 9, **caractérisée en ce que** l'ingrédient actif est sélectionné parmi des médicaments pharmaceutiques, des composés agrochimiques incluant des pesticides et des engrais, des biocides, des micronutriments, des agents antimicrobiens incluant des agents antifongiques et des agents antibactériens, et des mélanges de ceux-ci ; de préférence le biocide est sélectionné à partir du groupe consistant en des phénols, des phénols halogénés, des composés contenant de l'halogène, des composés libérant de l'halogène, des isothiazolinones, des composés contenant de l'aldéhyde, des composés libérant de l'aldéhyde, des biguanides, des sulfones, des thiocyanates, des pyrithiones, des antibiotiques tels que des antibiotiques β-lactam,
des sels d'ammonium quaternaire, des peroxydes, des perchlorates, des amides, des amines, des métaux lourds, des enzymes biocides, des polypeptides biocides, des azoles, des carbamates, des glyphosates, des sulphonamides et des mélanges de ceux-ci ; plus préférentiellement l'ingrédient actif est sélectionné parmi du L-carvone, du chloramphénicol, de la curcumine, du 2-phénylphénol, de la vanilline et des mélanges de ceux-ci ; le plus préférentiellement le composé actif est du chlorampénicol ou du 2-phénylphénol.

11. Système aqueux, pouvant être obtenu en mettant en contact de l'eau et un support particulaire chargé comprenant du carbonate de calcium ayant réagi en surface chargé d'un ingrédient actif, ledit ingrédient actif présentant une limite de solubilité dans l'eau inférieure à 10 g/l, mesurée à 20 °C et à 1 bar,
**caractérisée en ce que** le système aqueux comprend ledit ingrédient actif sous une forme dissoute, dans laquelle la concentration en masse de l'ingrédient actif dissous dans ledit système aqueux correspond à un état sursaturé, dans laquelle le carbonate de calcium ayant réagi en surface est un produit réactionnel de carbonate de calcium naturel (GNCC) ou de carbonate de calcium précipité (PCC) traité avec du CO₂ et un ou plusieurs donneurs d'ions H₃O⁺, dans laquelle le CO₂ est formé in situ par le traitement avec des donneurs d'ions H₃O⁺ et/ou est fourni à partir d'une source externe.

12. Procédé de préparation d'un système aqueux comprenant un ingrédient actif sous une forme dissoute, le procédé comprenant les étapes consistant à :
(a) fournir du carbonate de calcium ayant réagi en surface ;
(b) fournir un ingrédient actif présentant une limite de solubilité dans l'eau inférieure à 10 g/l, mesurée à 20 °c et à 1 bar ;
(c) charger le carbonate de calcium ayant réagi en surface fourni à l'étape (a) avec l'ingrédient actif fourni dans l'étape (b) pour obtenir un support particulaire chargé ;
(d) fournir de l'eau ;
(e) mettre en contact le support particulaire chargé obtenu à l'étape (c) et l'eau fournie à l'étape (d) ; et
(f) mélanger le support particulaire chargé et l'eau mis en contact dans l'étape (e) ; **caractérisé en ce que** la concentration en masse de l'ingrédient actif dissous dans ledit système aqueux correspond à un état sursaturé, dans laquelle le carbonate de calcium ayant réagi en surface est un produit réactionnel de carbonate de calcium naturel (GNCC) ou de carbonate de calcium précipité (PCC) traité avec du CO₂ et un ou plusieurs donneurs d'ions H₃O⁺, dans laquelle le CO₂ est formé in situ par le traitement avec des donneurs d'ions H₃O⁺ et/ou est fourni à partir d'une source externe.
